(19) Europäisches Patentamt
European Patent Office
Office européen des brevets

(11) EP 4 310 489 A1

(12) EUROPEAN PATENT APPLICATION

(43) Date of publication:
24.01.2024 Bulletin 2024/04

(51) International Patent Classification (IPC):
G01N 27/327 (2006.01)     G01N 33/543 (2006.01)

(21) Application number: 22186139.6

(52) Cooperative Patent Classification (CPC):
G01N 27/3275; G01N 33/5438

(22) Date of filing: 21.07.2022

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: ETH Zurich
8092 Zurich (CH)

(72) Inventors:
• DE MELLO, Andrew
8044 Zürich (CH)
• SHIH, Chih-Jen
8037 Zürich (CH)
• SUEA-NGAM, Akkapol
8093 Zürich (CH)
• BEZINGE, Léonard
1965 Saviese (CH)
• RICHARDS, Daniel
8165 Schöfflisdorf (CH)

(54) **ASSAY DEVICE FOR ELECTROCHEMICAL SENSING OF A SAMPLE FLUID**

(57) An assay device (1) for electrochemical sensing of a sample fluid comprises, at least one substrate (2), at least one electrode (3), and at least one channel (5). The channel (5) extends at least partially in the substrate (2) and is configured to receive the sample fluid. At least part of the electrode (3) is arranged in the channel (5) and is configured to detect at least one electrical property being associated with the sample fluid. The substrate (2), at least in the region of the channel (5), is porous. At least the part of the electrode (3) being arranged in the channel (5) is porous. The substrate (2) is configured to exert a capillary force onto the sample fluid in the channel (5) such that the sample fluid is flowing through the part of the electrode (3) being arranged in the channel (5).

FIG. 7a

EP 4 310 489 A1

**Description**

TECHNICAL FIELD

The present invention relates to an assay device for electrochemical sensing of a sample fluid according to claim 1 and to a method of manufacturing such an assay device according to claim 13.

PRIOR ART

Low-cost paper-based assay devices such as lateral flow assays are an essential tool for disease diagnostics. However, the currently available assay devices generally produce a colorimetric readout that typically provides semi-quantitative results. Quantification is paramount in diagnostics tests that compare results to a threshold, e.g. serology, or monitor trends, e.g. metabolic panel. Assay devices in the form of electrochemical platforms enable highly quantitative readout. Also, connected to a smartphone, they provide unambiguous results for self-testing use or field-deployable applications. The electrode fabrication on these paper-based devices remains challenging due to the rough and inhomogeneous nature of cellulose fibres. Moreover, electrodes in flow-based assays only quantify a fraction of the sample fluid due to their non-porous nature, and might, in certain format, obstruct the liquid flow. In addition, the manufacturing costs of electrochemical platforms are often prohibitive for single use applications. Electrode re-usability is generally not an option due to biofouling or the need of extensive washing steps.

Assay devices comprising capillary-driven electrochemical platforms on paper have been demonstrated but generally suffer from complex manufacturing, poor patternability, or incompatibility with flow-based assays due to the non-porous nature of the electrodes. The most common fabrication methods include screen printing, inkjet printing, thermoplastic molding and 3D printing. In all these cases, the formulation of the conductive ink or paste (composition, binder, viscosity) is tedious. Additionally, the electrodes cover the top surface of the cellulose paper, reducing the detection efficiency and closing the possibility of flow-through detection.

SUMMARY OF THE INVENTION

It is an object of the present invention to provide an assay device for electrochemical sensing of a sample fluid that allows electrochemical sensing at high efficiency and that can be manufactured at low cost.

This object is achieved with the assay device according to claim 1. In particular, an assay device for electrochemical sensing of a sample fluid is provided, wherein the assay device comprises at least one substrate, at least one electrode, and at least one channel. The substrate extends along a vertical direction of the assay device and along a horizontal direction of the assay device running perpendicularly to the vertical direction. The channel extends at least partially in the substrate and is configured to receive the sample fluid. At least part of the electrode is arranged in the channel and is configured to detect at least one electrical property being associated with the sample fluid. The substrate, at least in the region of the channel, is porous. Furthermore, at least the part of the electrode being arranged in the channel is porous. The substrate is configured to exert a capillary force onto the sample fluid in the channel such that the sample fluid is flowing through the part of the electrode being arranged in the channel.

The substrate being porous means that the substrate comprises pores.

A pore diameter of the pores in the substrate preferably is in the range of 0.003 micrometer to 400 micrometer, more preferably in the range of 0.1 micrometer to 100 micrometer, and particularly preferably in the range of 0.4 micrometer to 20 micrometer.

The electrode being porous means that the electrode comprises pores.

A pore diameter of the pores in the electrode preferably is in the range of 0.003 micrometer to 400 micrometer, more preferably in the range of 0.1 micrometer to 100 micrometer, and particularly preferably in the range of 0.4 micrometer to 20 micrometer. In other words, it is preferred that the pore diameter of the electrode is similar to or essentially corresponds to the pore diameter of the substrate.

Said pore diameter is determined according to the harmonized standard: USP <267> Porosimetry by Mercury Intrusion, see also https://www.usp.org/harmonization-standards/pdg/general-chapters/porosimetry-mercury-intrusion. That is, the pore diameter is determined as follows:

$$d_p = -\frac{4\,\sigma}{p}\,cos\theta,$$

where $p$ is the applied pressure, in Pascal,
$d_p$ is the pore diameter, in meter,

$\sigma$ is the surface tension of mercury, in Newton per meter, and

$\theta$ is the contact angle of mercury on the sample, in degree.

In the present invention, the surface tension of mercury ($\sigma$) was 0.485 Newton per meter and the contact angle $\theta$ corresponded to 130°.

Furthermore, the penetrometer stem volume was 0.412 millilitre, low pressure measurement were performed from 3.3 kPa to 345 kPa, and high pressure measurement were performed from 345 kPa to 225 MPa.

It is furthermore preferred that a porosity factor of the substrate is larger than zero. Additionally or alternatively, it is preferred that the porosity factor of the substrate is in the range of 0.4 to 0.85 and particularly preferably in the range of 0.5 to 0.75.

It is furthermore preferred that a porosity factor of the electrode essentially corresponds to the porosity factor of the substrate.

The porosity factor is defined as the ratio of the total volume of pores divided by the total volume of the substrate and the electrode, respectively.

The electrode is preferably associated with an electroactive porosity factor, wherein said electroactive porosity factor preferably is in the range of 3 to 10. In electrochemical systems such as the assay device according to the invention, an electroactive surface area of the electrode can be measured using cyclic voltammetry with a known redox probe. In the case of rough or porous electrode surfaces, this electroactive area is higher than the projected area. The ratio of the two is known as the electrochemical porosity factor. An electrochemical porosity factor of 1 will represent a planar electrode surface, whereas porous electrodes will have a factor > 1. The measurement method is well established and is outlined in detail with reference to the figures.

The capillary force being generated by the porous substrate on the sample fluid means that the substrate exhibits a capillary effect. The capillary effect occurs when the capillary forces, i.e. an adhesive force between the sample fluid and substrate, overcome the surface tension of the sample fluid, i.e. a cohesion force.

Moreover, the capillary force exerted by the substrate results in a capillary flow of the sample fluid in the channel. The capillary flow depends on the pore diameter as well as on the wettability, i.e. the water contact angle, of the assay device, in particular of the substrate and the electrode, see also further below.

The porosity of the substrate preferably results in the substrate being permeable to the sample fluid. Likewise, the porosity of the electrode preferably results in the substrate being permeable to the sample fluid. In other words, the sample fluid can flow through the porous substrate and through the porous electrode. That is, the electrode at least in the region of the channel can be said to behave as the porous substrate.

Furthermore, the porous substrate exhibiting a capillary force is preferably configured to drive a capillary flow of the sample fluid. Likewise, the porous electrode is preferably configured to exert a capillary force onto the sample in the fluid channel such that the sample fluid is flowing through the part of the electrode being arranged in the channel. Hence, the electrode is preferably configured to drive a capillary flow of the sample fluid as well.

The electrical property being associated with the sample fluid preferably is at least one of a a current, an electrical potential, an accumulated charge, or an impedance value.

The electrode can be arranged separately from the substrate. For instance, the electrode can be arranged above or below the substrate with respect to the vertical direction of the assay device.

However, it is likewise conceivable that the electrode is at least partially and preferably entirely arranged in the substrate.

In particular, it is preferred that the electrode is at least partially and preferably entirely integrated into the substrate. In other words, the electrode is preferably an integral part of the substrate. Again in other words, the electrode is preferably formed from as well as in the substrate. Hence, the electrode and the substrate are preferably a single-piece element. As such, the assay device of the present invention can be said to comprise a porous substrate with embedded or integrated electrodes and, in view of the capillary effect exhibited by the substrate, for capillary-driven assays.

The electrode is preferably generated by pyrolysis of the substrate, in particularly by laser-pyrolysis or by flash pyrolysis or by using a pyrolysis furnace. That is, the electrode is preferably generated by a thermal decomposition of the substrate. Hence, unlike an electrode that is for instance printed and as such added to the substrate, the electrode being generated by pyrolysis of the substrate corresponds to a (local) transformation of the substrate. In the laser-induced pyrolysis, the substrate act as the "reactant" of the pyrolysis reaction and is thus replaced by the electrode material.

The generation of the electrode by laser pyrolysis has the advantage of performing the pyrolysis reaction in a highly localized manner, thereby enabling the patterning of the shape of the electrode. However, other approaches are likewise conceivable, such as using a pyrolysis furnace or a lamp, preferably a UV lamp, for flash pyrolysis.

In order to generate the electrode by laser-pyrolysis, electromagnetic radiation, i.e. laser radiation, is irradiated onto the substrate, and wherein the electrode is formed at the incidence of the laser radiation on the substrate. The laser preferably is a commercially available laser, particularly preferably a $CO_2$ laser. The laser radiation preferably comprises a wavelength in the UV/VIS or IR region of the electromagnetic spectrum such as 100 nm to 20 micrometer. To this end it is particularly preferred to use a $CO_2$ laser at a wavelength of 10.6 micrometer. To this end it is particularly preferred that the electrode is generated by patterning. Said patterning is preferably achieved using a commercial $CO_2$ laser cutter, where the $CO_2$ laser is actuated using a gantry or a galvo system as it is known in the art.

In this case it is furthermore preferred that the substrate is treated with at least one fire retardant prior to the irradiation of the laser radiation in order to prevent a combustion of the substrate. Hence, the substrate preferably furthermore comprises at least one fire retardant. For instance, the substrate can be soaked in the fire retardant. A preferred fire retardant comprises or consists of at least one ammonium compound and/or at least one sulfate compound and/or at least one sulfamate compound and/or is halide free. The fire retardant particularly preferably is ammonium sulfamate or ammonium phosphate. Other fire retardants are however likewise conceivable. After the fire retardant is applied to the substrate, it is preferred to left the substrate dry.

After the generation of the electrode, preferably after the laser pyrolysis of the substrate, it is preferred to subject the electrode to a post-treatment. Said post-treatment serves the purpose of enhancing the hydrophilicity of the electrode that in turn enhances a flow of the sample fluid through the electrode being arranged in the channel. The post-treatment preferably corresponds to a plasma-treatment, in particular an oxygen-plasma or air-plasma treatment.

The substrate preferably comprise or consists of at least one paper-based compound and/or at least one polymeric membrane material.

The paper-based compound preferably comprises cellulose, in particular cellulose fibers. For instance, the substrate can be paper from Whatman CF3 that has a content of $\alpha$-cellulose of greater than 98 %.

The polymeric membrane material preferably is nitrocellulose or polysulfone.

The electrode preferably comprises or consists of at least one carbonaceous compound and/or at least one paper-templated metal.

The carbonaceous compound preferably comprises elemental carbon bonded through $sp^2$-hybridization. In other words, the carbonaceous compound preferably is a graphenic carbon material. For instance, preferred carbonaceous compounds comprise a graphenic nanomaterial of crystallites having lateral sizes of 4 nanometer to 5 nanometer and/or comprising 2 to 3 layers.

The electrode comprising or consisting of a paper-templated metal is preferably fabricated by the laser-induced reduction of a metal salt such as gold(III) chloride and/or palladium(II) nitrate.

The substrate is preferably electrically non-conductive.

The electrode is preferably electrically conductive and preferably furthermore has a sheet resistance in the range of 0.1 $\Omega$/sq to 100 k$\Omega$/sq, more preferably in the range of 0.1 $\Omega$/sq to 1 k$\Omega$/sq, and particularly preferably in the range of 0.1 $\Omega$/sq to 100 $\Omega$/sq. Said sheet resistance is measured according to the standard practice ASTM F1711-96 using the 4-probe method in square configuration and a 2 millimeter probe spacing.

At least the part of the electrode being arranged in the channel is preferably hydrophilic. It is furthermore preferred that at least the part of the electrode being arranged in the channel has a water contact angle in the range of 90° or less and particularly preferably in the range of 0°-60°.

A part of the electrode is preferably arranged outside of the channel, and wherein said part of the electrode preferably is hydrophobic. The part of the electrode being arranged outside of the channel preferably has a contact angle of greater than 90° and particularly preferably in the range of 110°-130°.

In the region of the channel, the substrate is preferably hydrophilic. It is furthermore preferred that the substrate, in the region of the channel, has a water contact angle in the range of 90° or less and particularly preferably in the range of 0°-60°.

In a region outside of the channel, the substrate is preferably hydrophobic. Additionally, it is preferred that the substrate in the region outside of the channel has a water contact angle of greater than 90° and particularly preferably in the range of 110°-130°.

That is to say, the electrode and the substrate preferably have a wetting behaviour that is essentially the same. In particular, the part of the electrode being arranged in the channel preferably exhibits a fast wetting being comparable to the wetting of the substrate and vice versa.

The assay device preferably further comprises at least one coating. The coating is preferably at least partially arranged on and/or in the substrate. Additionally or alternatively, the coating is preferably at least partially arranged on and/or in the electrode.

It is furthermore preferred that the coating at least partially delimits the channel.

Furthermore, the coating is preferably hydrophobic and/or has a melting temperature of 150° or less and/or comprises or consists of wax, preferably paraffin.

The coating being arranged on the substrate and/or on the electrode means that the coating is at least partially arranged on a surface of the substrate and/or on a surface of the electrode.

The coating being arranged in the substrate and/or in the electrode means that the coating has at least partially entered or penetrated into the substrate or the electrode. For instance, in the event of the coating being meltable the melted coating can flow into the substrate or into the electrode, whereby it is contained within the substrate or the electrode.

Since the coating is preferably hydrophobic, it can render the substrate and the electrode hydrophobic. For example, in the event of the substrate being paper and as such hydrophilic, the application of the hydrophobic coating renders the paper on its outside and/or in its inside hydrophobic. As such, the application of the coating in a targeted manner allows the specific creation of hydrophobic and hydrophilic regions on and/or in the substrate as well as on and/or in the electrode.

To this end, it is particularly preferred that the coating can be melted at low temperatures such as at 150° C or less, preferably at 110° C or less. Moreover, a preferred coating is wax, such as paraffin. However, other coatings are likewise conceivable.

It is furthermore preferred that the coating is electrically non-conductive.

In this respect it is noted that the electrode with or without coating is preferably electrically conductive. However, the substrate with or without coating is preferably electrically non-conductive. In particular, the electrically conductive properties of the electrode are preferably unaffected when being provided in a fully in coated e.g. waxed region or at a wax interface.

The coating preferably furthermore comprises a recess in the form of a channel outline. Hence, when applied to the substrate, the channel is at least partially delimited by the coating. Stated differently, the channel extends within the recess of the coating. As such, the assay device is capable of maintaining a sample fluid within a channel of a hydrophilic substrate such as the paper substrate. Namely, without the hydrophobic coating delimiting the channel, the sample fluid would not remain within the channel but flow into other regions of the hydrophilic substrate.

The substrate preferably comprises a thickness along the vertical direction of the assay device being preferably in the range of 10 micrometer to 5000 micrometer, more preferably in the range of 50 micrometer to 1000 micrometer, and particularly preferably in the range of 200 micrometer to 400 micrometer. Additionally or alternatively, the substrate preferably comprises a thickness along the vertical direction of the assay device being 500 micrometer or less, particularly preferably being 300 micrometer or less.

The thickness of the substrate along the vertical direction is important with respect to its fire resistance as well as in view of getting hydrophobic. Namely, in the event that the substrate is too thin, it can burn or become too brittle upon the irradiation of electromagnetic radiation during the electrode generation by laser pyrolysis. However, if the substrate is too thick, the coating such as the wax might still be able to penetrate, but not deeply enough to cover an entire cross-section of the substrate. In particular, the coating preferably covers an entire cross-section of the substrate in order to delimit a tight channel for the fluid sample to flow in. For instance, a preferred substrate corresponds to a 300 micrometer thick paper, wherein the coating in the form of was has penetrated by 150 micrometer to 175 micrometer into the substrate.

To this end, it is furthermore preferred that the substrate is provided in the form of a layer, i.e. the substrate preferably is a substrate layer. Hence, the substrate comprising the electrode can be seen as an electrode layer or a functional layer that extends within a plane being perpendicular to the vertical direction. As will be mentioned further below, the assay device can comprise two or more functional layers or electrode layers. Said two or more functional layers or electrode layers are preferably arranged above one another, i.e. stacked, with respect to the vertical direction of the assay device.

In one aspect, the channel preferably extends along the vertical direction of the assay device, and wherein the substrate is configured to exert the capillary force onto the sample fluid in the channel such that the sample fluid is flowing along a vertical flowing direction running parallel to the vertical direction of the assay device. In fact, the assay device preferably comprises two or more functional layers or electrode layers being stacked and as mentioned earlier, and wherein the channel preferably extends into these two or more substrate layers, functional layers or electrode layers, respectively, when seen along the vertical direction of the assay device.

That is, the assay device can be a so-called vertical-flow assay device, wherein the sample fluid flows along the vertical direction of the assay device.

To this end, it is preferred that the channel extends at least partially and preferably entirely through the substrate along the vertical direction of the assay device. Consequently, a length of the channel along the vertical direction preferably corresponds to the thickness of the substrate, in particular to the sum of the thicknesses of all substrate layers, functional layers or electrode layers, respectively, being stacked, along the vertical direction of the assay device, see above.

In this context, it is noted that the channel preferably has a circular shape when seen in cross-section along the vertical direction of the assay device. In other words, the channel preferably has a tubular shape along the vertical direction of the assay device. A preferred diameter of the channel is in the range of 0.1 millimeter to 20 millimeter such as 7.5 millimeter. However, channels of other shapes and dimensions are likewise conceivable.

It is furthermore preferred that the assay device comprises at least one sample pad. The sample pad is preferably arranged before the substrate when seen along the vertical direction of the assay device. In use condition of the assay device, the sample pad can be said to be arranged above the substrate. It is furthermore preferred that the sample pad is porous and/or comprises or consists of at least one paper-based compound, preferably cellulose. The sample pad preferably serves the purpose of protecting components of the assay device being arranged after the sample pad when seen along the vertical direction of the assay device. In use condition of the assay device, the sample pad can be said to protect the components of the assay device being arranged below the sample pad. Additionally or alternatively, the sample pad preferably serves the purpose of homogenising the wetting of the channel across the whole cross-section. As such, the sample pad preferably is associated with a fast wetting. Furthermore, the sample pad preferably has a low volume retention and as such a small thickness with respect to the vertical direction of the assay device in order minimize the undetected volume. A preferred thickness of the sample pad along the vertical direction of the assay assembly is 100 micrometer or less. Furthermore, the sample pad preferably has a low protein binding capacity.

In addition or in the alternative, the channel preferably extends along the horizontal direction of the assay device, and wherein the substrate is configured to exert the capillary force onto the sample fluid in the channel such that the sample fluid is flowing along a horizontal flowing direction running parallel to the horizontal direction of the assay device.

Hence, in another aspect, the channel preferably extends along the horizontal direction of the assay device, however not along the vertical direction of the assay device. That is, the assay device can be a so-called lateral-flow assay device, wherein the sample fluid flows along the horizontal direction of the assay device.

In another aspect, the channel preferably extends along the vertical direction of the assay device as well as along the horizontal direction of the assay device. That is, the channel can be a combination of a vertical channel and a horizontal channel, i.e. the channel can be a three-dimensional channel comprising vertical and lateral channel sections. As such, the assay device can be a three-dimensional assay device. Said three-dimensional assay device preferably comprises multiple functional layers or electrode layers that are stacked.

It is preferred that the channel extends only partially or entirely through the substrate along the vertical direction of the assay device.

A length by which the channel extends into the substrate when seen along the vertical direction of the assay device preferably corresponds to 50 % to 100% of the thickness of the substrate along the vertical direction of the assay device. For instance, in the event of a substrate having a thickness of 300 micrometer, the length by which the channel extends into the substrate is preferably between 150 micrometer to 300 micrometer with respect to the vertical direction of the assay device.

A preferred length of the channel along the horizontal direction of the assay device preferably is between 5 millimeter to 40 millimeter. If it is too short, it does not leave enough space for the electrode. If it is too long, the capillary-driven flow of the sample fluid becomes very slow.

It is furthermore preferred that the channel has the shape of an elongated recess when seen along the horizontal direction of the assay device.

A horizontal width or cross-section of the channel along the horizontal direction preferably is in the range of 0.5 millimeter to 10 millimeter, more preferably in the range of 2 millimeter to 5 mm. If it is too narrow, it provides a small detection volume. If it is too wide, it requires a high usage of reagent.

The assay device preferably further comprises at least one buffer pad, wherein the buffer pad is at least partially arranged in contact with the channel and is configured to establish a preferably steady flow of a buffer solution from a buffer reservoir of the assay device across the substrate and preferably to an absorbent pad, see further below. As such, the assay device is preferably configured for flow injection analysis, i.e. the sequential analysis of multiple sample fluids spiked into a continuously running buffer solution.

The buffer pad preferably serves the purpose of providing a wetting interface between the buffer reservoir and the channel.

The buffer pad is preferably a porous and/or a paper-based compound such as cellulose. For instance, a preferred buffer pad is a thick cellulose, for instance cellulose with a thickness of 1.5 millimeter with respect to the vertical direction of the assay device.

The buffer solution preferably has buffering properties that enable to keep a constant chemical environment such as an ionic strength or pH-value of the sample fluid in order to ensure a detection of the electrical property being unaffected by the nature of a sample spiked into the flow.

A preferred buffer solution comprises a buffer salt (constant pH) and a surfactant and blocking agent (protein, polymer) to reduce non-specific binding of the sample fluid.

To this end it is conceivable that the buffer solution is provided in the buffer reservoir and for instance sealed with a seal at a manufacturing level of the assay device. However, it is likewise conceivable that the assay device is manufactured without any buffer solution in the buffer reservoir but is supplied separately to be filled in the buffer reservoir by the user.

In any case it is preferred that the assay device further comprises at least one absorbent pad being configured to absorb the sample fluid. The absorbent pad can be arranged before and/or after the substrate when seen along the vertical direction. In fact, in the event of the channel extending along the vertical direction, it is preferred that at least one absorbent pad is arranged after the substrate when seen along the vertical direction of the assay device. In the event of the channel extending along the horizontal direction of the assay device, it is preferred that at least one absorbent pad is arranged before the substrate when seen along vertical direction of the assay device. In the event of the channel extending along the vertical and the horizontal direction of the assay device, it is preferred that at least one absorbent pad is arranged before and/or after the substrate when seen along the vertical direction of the assay device.

Furthermore, the absorbent pad can be arranged partially or entirely congruent with the channel, in particular with the part of the electrode being arranged in the channel, when seen along the vertical direction. For instance, in the event of the channel extending along the vertical direction of the assay device it is preferred that the absorbent pad is arranged at a same horizontal position as the channel. In this regard, it is furthermore preferred that a cross-section of the absorbent pad equals or is larger than a cross-section of the channel along the horizontal direction. In the event of the channel extending along the horizontal direction of the assay device it is preferred that the absorbent pad is arranged at least regionally horizontally offset from the channel. In particular, it is preferred that the absorbent pad is arranged in an end region of the channel when viewed downstream of the horizontal flowing direction.

The absorbent pad serves the purpose of absorbing the sample fluid that exits the channel along the vertical direction and/or along the horizontal direction of the assay device in order to drive a flow, in particular a capillary flow, of the sample fluid along the channel.

The absorbent pad preferably is hydrophilic and has a high absorption capacity, i.e. the absorbent pad preferably is porous and is furthermore configured to exhibit a capillary effect. The absorbent pad preferably comprises or consists of at least one paper-based compound, particularly preferably cellulose.

To this end it is conceivable to stack two or more absorbent pads above one another with respect to the vertical direction of the assay device. For example, an absorbent pad in the form of a cellulose layer with a thickness of 1.5 mm along the vertical direction of the assay device has a water absorption capacity of 136 microliter per $cm^2$, compared to 35 microliter per $cm^2$ of the substrate in the form of the 300 micrometer cellulose layer.

The assay device preferably further comprises at least one capture zone comprising capture molecules being configured to specifically capture at least one target property being associated with the sample fluid. Examples of conceivable capture molecules are proteins, DNA, RNA, virus, bacteria, cells, polymers such as molecular-imprinted polymers, small molecules or synthetic derivative thereof such as peptide nucleic acids. Examples of proteins are antibodies, nanobodies, antigens, enzymes, etc.

The capture zone can be provided as a capture pad being functionalized with the capture molecules. However, the capture zone can likewise be provided by the electrode being functionalized with the capture molecules.

The capture zone allows a detection of the electrical property using a labelled approach, wherein the electrical property is generated by the label.

However, it is likewise conceivable to detect the electrical property in a label-free approach, wherein the electrical property is directly detected, for instance by directly detecting binding events of the sample fluid e.g. via impedance measurement.

The capture zone in the form of the functionalized pad can be provided as a nitrocellulose layer that is furthermore functionalized, for instance with immobilized proteins such as antigens or antibodies that are specific for the target property. Immobilization can be performed by drop casting or inkjet printing of a protein solution comprising the proteins onto the nitrocellulose layer.

The functionalization of the electrode preferably occurs in at least one functionalization step that preferably takes place after the generation of the electrode, in particular the electrode layer, via wet chemistry and using a chemical linker to bind the capture molecules such as biomolecules covalently or via electrostatic interaction to a surface of the electrode. In particular, the electrode is preferably prepared as described earlier from pyrolysis of cellulose, for instance, and is thereafter functionalized chemically. To this end it is particularly preferred that the capture zone in the form of the functionalized electrode corresponds to an electrode made from pyrolyzed cellulose and that is furthermore functionalized with immobilized proteins such as antigens or antibodies.

That is, the assay device comprising the capture zone is preferably configured to perform a labelled detection, wherein the affinity of the capture zone such as the antibody/antigen affinity binding is leveraged to label a target property or target of interest (antigen, antibody, small molecule, virus). The label is generally electroactive or generates a redox probe (e.g. enzyme, nanocatalyst) or the like, i.e. an electrical property being associated with the label and being detected by the electrode. Hence, the assay device can be an immunoassay device.

However, and as mentioned earlier, it is likewise conceivable that the assay device is configured without a capture zone to perform an unlabelled detection, in which the target property or target in the sample fluid is electroactive (e.g. some small molecules, metal ions) or can take part in a reaction that produces an electrochemically detectable product (e.g. enzyme detection, small molecule enzymatic detection).

The electrode preferably is a so-called working electrode, at which the detection of the electrical property being associated with the sample fluid occurs. That is, the detection of the electrical property being associated with the sample fluid preferably corresponds to a so-called electrochemical detection. Preferably, the assay device comprises at least two electrodes, said working electrode and at least one counter electrode, and wherein an electrical potential or current can flow between the working electrode and the counter electrode, i.e. an electrical potential or current is applicable and/or measurable between the working electrode and the counter electrode. It is however likewise conceivable that the assay device comprises three electrodes, namely said working electrode, the counter electrode and additionally also a so-called reference electrode, so that the electrical potentials being applied and/or measured at the working electrode are established with respect to the potential of the reference electrode. If no reference electrode is present, the electrical potentials are applied and/or measured between the working and the counter electrode.

In other words, the working electrode combined with a counter electrode and a reference electrode preferably forms a three-electrode system and the basis of a so-called set of electrodes being configured for electrochemical detection.

The reference electrode can be coated with a reference material such as silver and/or silver chloride. However, it is likewise conceivable that the reference electrode is uncoated and serves as a pseudo-reference. A pseudo-reference electrode does not have a stable and known standard electrical potential, but is able to maintain a constant potential over the course of the measurement. In this case, the electrical potentials that are measured and/or applied cannot be reported against a standard value (e.g. standard hydrogen potential), but are reported against the pseudo-reference electrode potential. A true reference electrode is preferred if the values of the electrical potential are compared with other systems.

The electrodes are preferably arranged in the assay device such that the reference electrode is close to the working electrode, for instance within a distance of 0.1 millimeter to 2 millimeter. Furthermore, it is preferred that the counter electrode has a maximal surface area with respect to the working electrode to ensure fast counter reaction and avoid any migration limitation.

At least the working electrode is manufactured as described earlier. Any further electrodes such as the counter electrode, the reference electrode, or the pseudo-reference electrode can be manufactured as described earlier as well or differently. For instance, the further electrodes can be pyrolyzed or not and be metal wires instead, for instance. In this regard it is furthermore conceivable that the further electrodes are made of a different material, such as the counter electrode being a platinum wire and the reference electrode being a silver wire, etc.

Furthermore the electrode and one or more further electrodes such as the counter electrode, the reference electrode or the pseudo-reference electrode can be arranged in particular embedded on the same substrate. However, it is likewise conceivable that one or more of these further electrodes are arranged on different pads or layers of the assay device, for instance on different substrate layers or are part of a different functional layer or electrode layer.

The assay device preferably furthermore comprises at least one evaluation device being configured to evaluate the electrical property being detected by the electrode, see below.

The assay device preferably further comprises a casing, and wherein the casing at least partially encases the substrate, the electrode and the channel. Furthermore, the casing can comprise at least one sample inlet through which the sample fluid is insertable into the channel. Additionally or alternatively, the casing can comprise at least one electrode contact being in electrical connection with the electrode and being configured to transmit an electrical signal of the electrode being associated with the detected electrical property to an evaluation device. Additionally or alternatively, the casing can comprise at least one buffer reservoir being configured to receive a buffer solution.

The casing preferably is a multi-part piece that comprises at least a top casing and a bottom casing that are arranged opposite to one another with respect to the horizontal direction. The casing preferably is at least one of non-wicking, non-porous, inert, and has a low protein absorption. Furthermore, the casing preferably comprises or consists of plastic such as polyacrylate or polyethylene and/or of plastified cardboard. Various ways of manufacturing the casing are conceivable such as injection molding or 3D printing.

It is furthermore preferred that the top casing and the bottom casing face towards an outside, i.e. the top casing and the bottom casing are preferably the outermost components of the assay device.

The top and bottom casing preferably encase the further components of the assay device such as the sample pad, the absorbent pad, and the buffer pad. The sample inlet and/or the electrical contact and/or the buffer reservoir are preferably provided on the top casing.

The electrode contact preferably serves the purpose of transmitting the electrical property being associated with the sample fluid and being detected by the electrode to an evaluation device being configured to evaluate the detected electrical property.

The electrode contact is preferably in electrical connection with the electrode via one or more metallic pads or the like. The electrode contact is preferably furthermore in connection with at least one evaluation device for read-out. Said connection can take different forms. For instance, large spring contacts for convenience and connectability to external plugs could be used. However, it is likewise conceivable to have a connection by means of soldered wires or a direct contact with the pads of an evaluation device in the form of a PCB or microcontroller for example.

Said evaluation device can be part of the assay device. For instance, the assay device could comprise an evaluation device in the form of a microprocessor that is embedded in the assay device and which is in communication with the electrode in order to receive the detected electrical property. For instance, the microprocessor could be embedded into the casing and its evaluation result could be displayed via a display being provided in the assay device as well or being transmitted preferably wirelessly to a remote display device. That is, the assay device can comprise a display device being arranged on the assay device, preferably in the casing and thus visible to a user, ore remotely from the assay device in order to display the detected electrical property and/or an evaluation result of the evaluation device. However, the evaluation device can likewise be arranged remote from the assay device. For instance, in order to evaluate the detected electrical property an electrical device capable of performing voltammetry, chronoamperometry or potentiometry, or any other commonly used electrochemical method could be attached to the electrode contact. Also in the case of a remote evaluation device it is conceivable that the assay device comprises a display device as mentioned earlier.

In any case it is particularly preferred that the assay device comprises the at least one reference electrode mentioned earlier, and wherein the evaluation device is configured to apply a voltage or current to the set of electrodes and to record the response.

That is, the evaluation device is preferably a potentiostat and/or galvanostat. The evaluation device is preferably configured to control a voltage or current between certain electrodes according to the desired method of detection, e.g. square wave voltammetry, and to record the voltages or currents between certain electrodes.

The sample fluid is preferably insulated from the electrical pad and further electrical components of the assay device such as the metallic pads. Said insulation is preferably provided by the coating.

It is noted here that the assay device can comprise only one substrate and/or one electrode and/or one channel and/or one sample pad, etc. or two or more substrates and/or two or more electrodes and/or two or more channels and/or two or more sample pads, etc. Hence, any explanations provided with regard to one of these components likewise apply to the provision of two or more of these components.

For example, the assay device could comprise multiple channels and a single set or multiple set of electrodes, i.e. the electrode ("working electrode"), the reference electrode, and the counter electrode, see above. However, it is likewise conceivable that the assay device comprises multiple sets of electrodes and a single channel. This latter case is particularly preferred in the event of the assay device being a vertical-flow assay device, wherein the sample fluid flows along the vertical direction of the assay device. In this case, it is conceivable to provide several sets of electrodes that are preferably arranged above one another or stacked along the vertical direction of the assay device, wherein an advantage of this assay device is its compact design. These designs allow many applications of the assay device, such as a sensing of multiple targets, various target concentration ranges, internal calibration or integrated negative/positive controls, the latter being very important for immunoassays.

In another aspect, a method of manufacturing an assay device for electrochemical sensing of a sample fluid, preferably the assay device as described above, is provided. The method comprises the steps of i) providing at least one substrate, ii) providing at least one electrode, and iii) providing at least one channel. The substrate extends along a vertical direction of the assay device and along a horizontal direction of the assay device running perpendicularly to the vertical direction. The channel extends at least partially through the substrate and is configured to receive the sample fluid. At least part of the electrode is arranged in the channel and is configured to detect at least one electrical property being associated with the sample fluid. The substrate at least in the region of the channel is porous. At least the part of the electrode being arranged in the channel is porous. The substrate is configured to exert a capillary force onto the sample fluid in the channel such that the sample fluid is flowing through the part of the electrode being arranged in the channel.

Any explanations made herein regarding the assay device per se preferably likewise apply to the method of producing the assay device and vice versa.

The electrode is preferably integrally formed in the substrate by pyrolysis of the substrate, in particular by laser pyrolysis or by flash pyrolysis or by using a pyrolysis furnace. For instance, and as mentioned earlier, the exposure of the substrate to electromagnetic radiation such as laser radiation results in the formation of the electrode. That is, in the event of the substrate being paper, the irradiation of laser radiation forms a graphenic material, which serves as the electrode in the assay device.

It is furthermore preferred that the electrode once being generated is treated with a plasma, in particular with an oxygen-plasma or an air plasma. To this end it is particularly preferred that the electrode is subject to oxygen or air at a pressure in the range of 0.5 mbar to 5 mbar, preferably of 1 mbar, and/or at a power in the range of 1 watt to 200 watt, preferably in the range of 5 watt to 100 watt, and/or during a time period of 5 seconds to 600 seconds, preferably of 90 seconds.

The treatment of the electrode by an oxygen plasma or air plasma increases the hydrophilicity of the electrode and induces a capillary flow of the sample fluid through the electrode that is similar to that of a substrate in the form of cellulose paper, for instance. Said treatment of the electrode is preferably performed after its generation in the substrate and before the coating is applied.

Hence, at least one coating is preferably provided, and wherein a recess in the form of a channel outline is removed from the coating preferably by irradiating laser radiation onto the coating.

The coating is preferably applied on and/or in the substrate after the recess in the form of the channel outline is formed. Additionally or alternatively, the coating is preferably applied on and/or in the electrode after the recess in the form of the channel outline is formed. It is furthermore preferred that the coating is applied by lamination.

That is, it is preferred to form the electrode in the substrate in one step and to form the recess in the form of the channel outline in the coating in another step. Thereafter, the coating comprising the recess in the form of the channel outline is preferably applied to the substrate comprising the electrode.

The recess in the shape of the channel outline in the coating is preferably formed by cutting, in particular by laser cutting. Said cutting can be seen as cutting off the hydrophilic parts to be formed in the assay device.

The coating preferably together with the recess in the form of the channel outline is preferably applied to the substrate and/or the electrode by lamination. It is furthermore preferred that the coating is melted during lamination, whereby it can enter or penetrate into the substrate and/or the electrode. Hence, the coating is particularly preferably applied to the substrate and/or the electrode by hot lamination. It is furthermore preferred that the coating is arranged on a carrier prior to its lamination to the substrate and the electrode, respectively. For example, the coating such as wax can be printed on a carrier such as a plastic film, for instance a thin polyester film, followed by cutting the recess with a laser cutter. The wax is then transferred onto and/or into the porous paper/electrode via lamination. It should be noted that the wax can be applied via a wax printer as well as with any wax-coated film.

It is furthermore preferred that the coating is applied on an upper side and a lower side of the substrate and/or the electrode in order to ensure that the coating can penetrate entirely through the substrate and/or the electrode with respect to the vertical direction of the assay device. For instance, a coating in the form of wax can penetrate 150-175 micrometer into a substrate in the form of 300 micrometer-thick paper, which is why it is preferred to laminate wax on both sides of the paper to achieve full waxing of the hydrophobic regions of the substrate. In this regard it is noted that the coating being applied on the upper side and the lower side of the substrate and/or the electrode can be applied in a pattern being the same or different. Coatings in the form of different patterns are preferred in the event of asymmetric channels.

The lamination is preferably performed with a lamination device as it is known in the state of the art.

It is furthermore to perform the lamination while pressure is applied between the two rollers of the lamination device. Said pressure is preferably such that good contact is established between the coating such as the wax and the substrate such as the paper, but low enough in order not to damange the electrodes.

The lamination temperature is preferably chosen in accordance with the coating and its melting temperature. For instance, in the event of the coating being wax, the lamination temperature is preferably 110 °C. Moreover, one or several such as 3 lamination passes can be performed in order to achieve full penetration of the coating, and wherein a lamination speed is preferably about 1 cm/s.

In addition, the further components of the assay device such as the absorbent pad or the sample pad are preferably cut into a shape that matches the shape of the substrate, the channel or the electrode from larger sheets such as larger sheets of cellulose/nitrocellulose. Thereafter, they are preferably assembled together with the other components of the assay device.

Hence, in summary it can be said that the assay device according to the invention enables a flow-based assay such as a bioassay for biosensing, for instance a capture immunoassay or for continuous detection, or a chemical assay for chemical sensing in lateral, vertical or three-dimensional flow format. The assay device can be used in in-vitro diagnostics and rapid tests, for instance. As outlined above, the assay device is an electrochemical platform with integrated or embedded porous electrodes that can be fabricated via pyrolysis, in particular laser pyrolysis, in a porous substrate such as a paper-based substrate. The wetting characteristics of a laser-pyrolyzed paper-based substrate such as cellulose have never been investigated so far and strongly differ from the well-studied pyrolysis of polyimide. The use of laser-pyrolyzed porous substrates such as cellulose in capillary-driven bioassay opens up new designs that are not possible with other electrode fabrication techniques. The assay device and its manufacturing according to the invention provide several advantages over the prior art. For instance, no specialized tools are required for the manufacturing of the assay device. Instead, the assay device can be fabricated using tools that are widely available such as a commercially available $CO_2$ laser for the laser-pyrolysis and a hotplate for the lamination of the coating to the substrate suffice. As such, the assay device can be manufactured at low-cost and scalable fabrication. Moreover, the assay device, in particular the arrangement and/or design of the channel and electrode as well as the presence or absence of hydrophobic, hydrophilic, electrically conductive or electrically non-conductive regions are on-demand, wherein no mask or stencil are required. That is, the assay devices according to the invention benefit from simplified and unique device architectures. Moreover, the porous nature of the electrode provides a large electroactive area. In the vertical flow format, the electrode can be sandwiched in the middle of a stack of pads, which is the first demonstration of such an assay device. For an immunoassay, a capture zone such as a nitrocellulose layer can simply be added above the electrode, thereby greatly reducing the device complexity. Furthermore, the vertical flow enables faster running time and higher efficiency of the electrode due to the improved wetting. In the lateral flow format, a continuous detection of multiple samples is achieved with high efficiency, wherein an entirety of the sample fluid can flow through the electrode.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

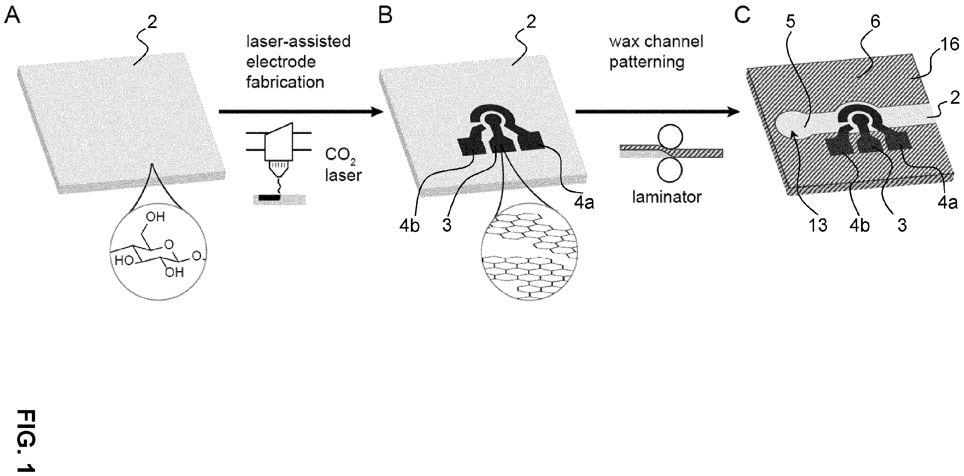

Fig. 1 shows a schematics of the method of manufacturing a substrate comprising a channel and an electrode of an assay device according to the invention. Starting from a substrate in the form of a cellulose paper layer (A), pre-treated with a fire retardant solution, the electrode is created and patterned (B) via laser-induced pyrolysis using a commercial $CO_2$ laser. A hydrophobic wax pattern is then transferred onto the paper via lamination to create the fluidic channel (C). The resulting functional layer thus consists of a combination of electrode systems and fluidic channels patterned independently from one another;

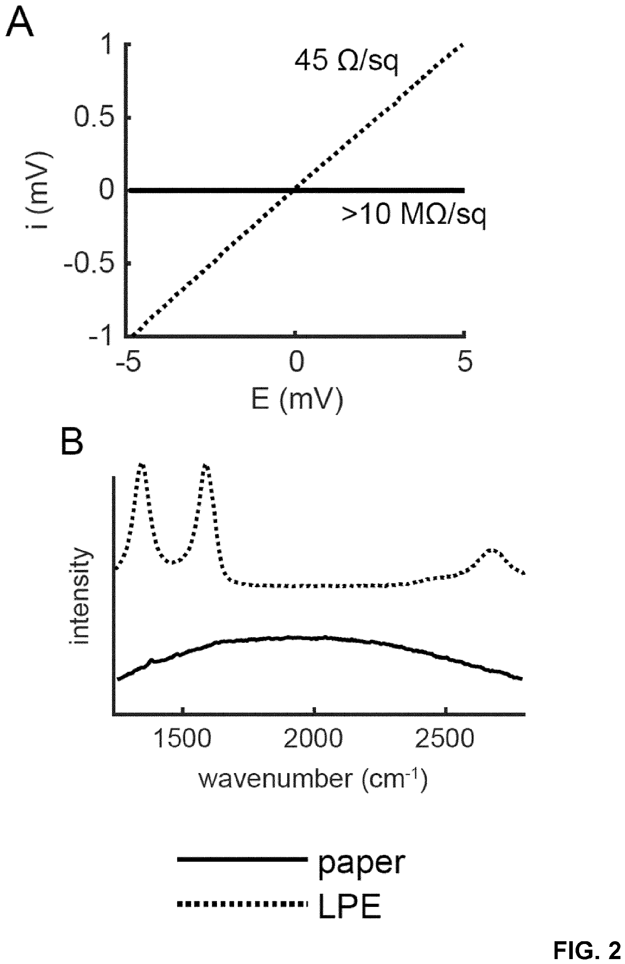

Fig. 2 shows how the pyrolysis process transforms the non-conductive cellulose paper into a graphenic conductive material. (A) Sheet resistance measurement of paper and the laser-pyrolyzed electrode (LPE) using the four-probe method. (B) Raman spectra of paper and LPE shows evidence of graphenic carbon formation (few layers graphene or "nanographite" domains);

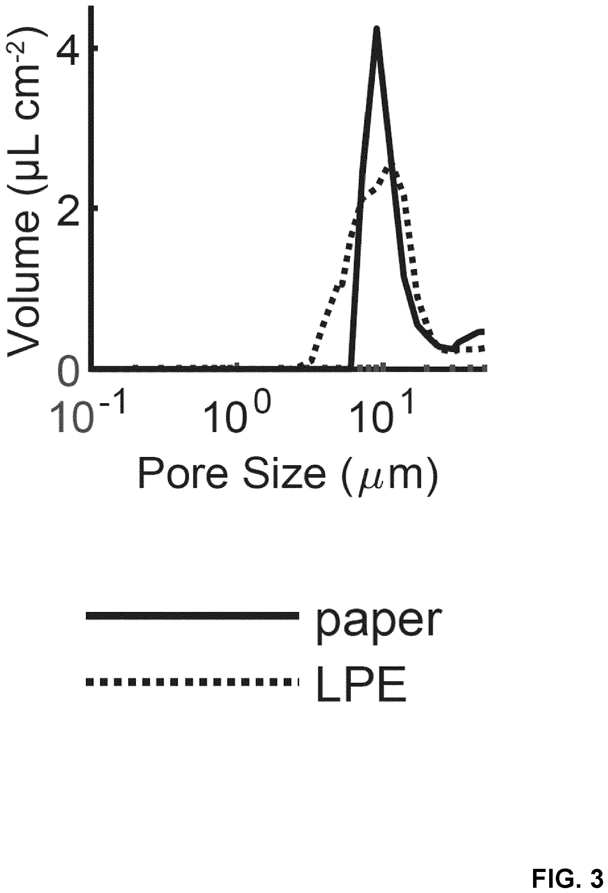

Fig. 3 shows porosity measurements of paper and LPE material using mercury porosimetry. Despite the harsh nature of the pyrolysis process, the resulting material keeps the porous nature of cellulose paper with a pore diameter of 11 $\mu$m;

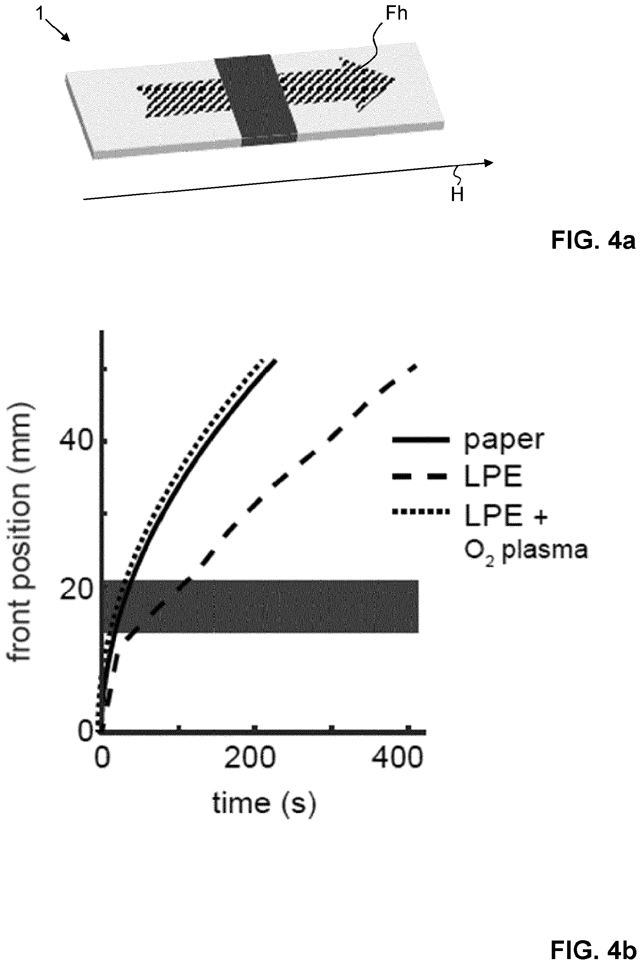

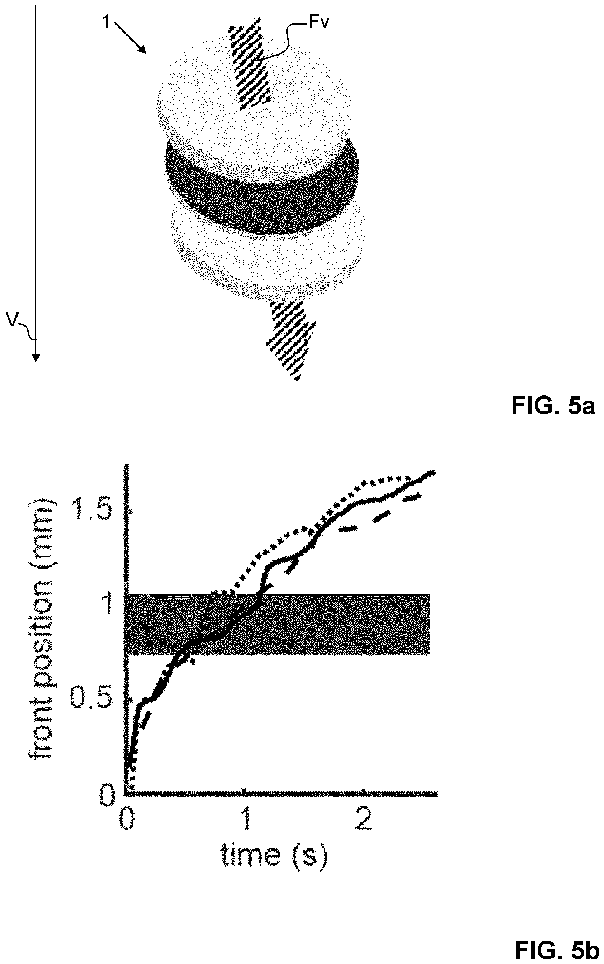

Fig. 4a-Fig. 5b show capillary flow measurements using real-time imaging of a dye solution wicking the paper substrates. The capillary flow, according to the Washburn equation depends on two key parameters: the pore diameter and wettability (water contact angle) of the material. Two configurations are compared: lateral flow format, with the front moving in-plane along the paper sheet substrate (figures 4a and 4b), and vertical flow format, where several layers are stacked and the flow is measured along the cross section of the stack (figures 5a and 5b). In lateral flow, the deeper layers of the LPE material exhibits hydrophobic characteristics and slow down the capillary flow. Treatment of the LPE by oxygen plasma increases hydrophilicity and induces capillary flow similar to that of cellulose paper. In the case of vertical flow, the top layer of the LPE shows great hydrophilicity with capillarity properties similar to cellulose even without oxygen plasma treatment. The black boxes in the front profile measurement represent the position of the electrode;

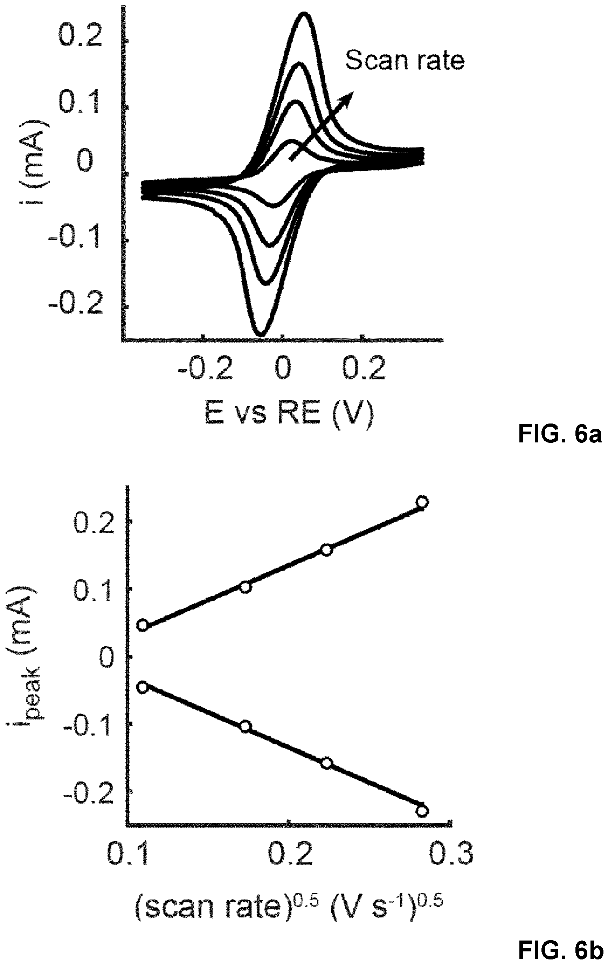

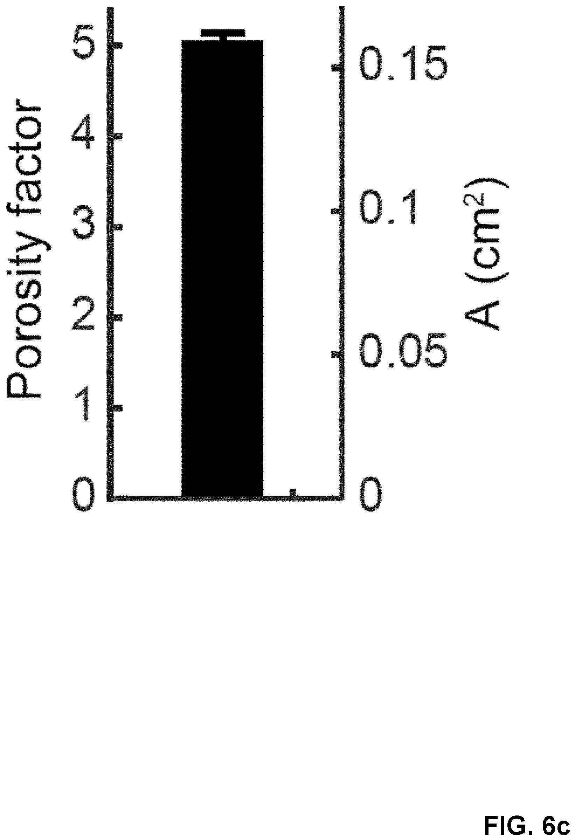

Fig. 6a-Fig. 6c show the electrochemical characterization of an assay device with a set of three electrodes made of LPE material, using a solution of 10 mM $[Fe(CN)_6]^{3-/4-}$ (a standard redox probe) in 1 M KCl. (A) Cyclic voltammetry at increasing scan rate (12, 30, 50, 80 mV/s). Potentials are reported with respect to the pseudo-reference electrode. The peak current are represented in the Randles-Sevcik plot (B), and the electroactive area (C) derived from the slope of the fitted line. Compared to the projected surface area, the LPE material exhibit high electrochemical porosity, with an electrochemical porosity factor of 5;

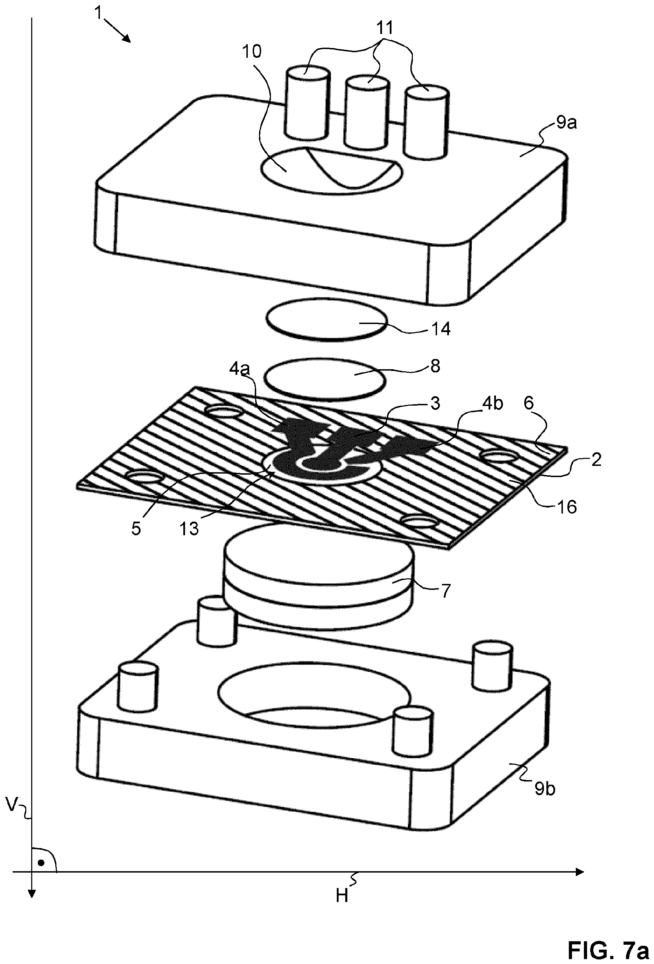

Fig. 7a show an exploded view of the assay device in the form of a vertical flow assay device;

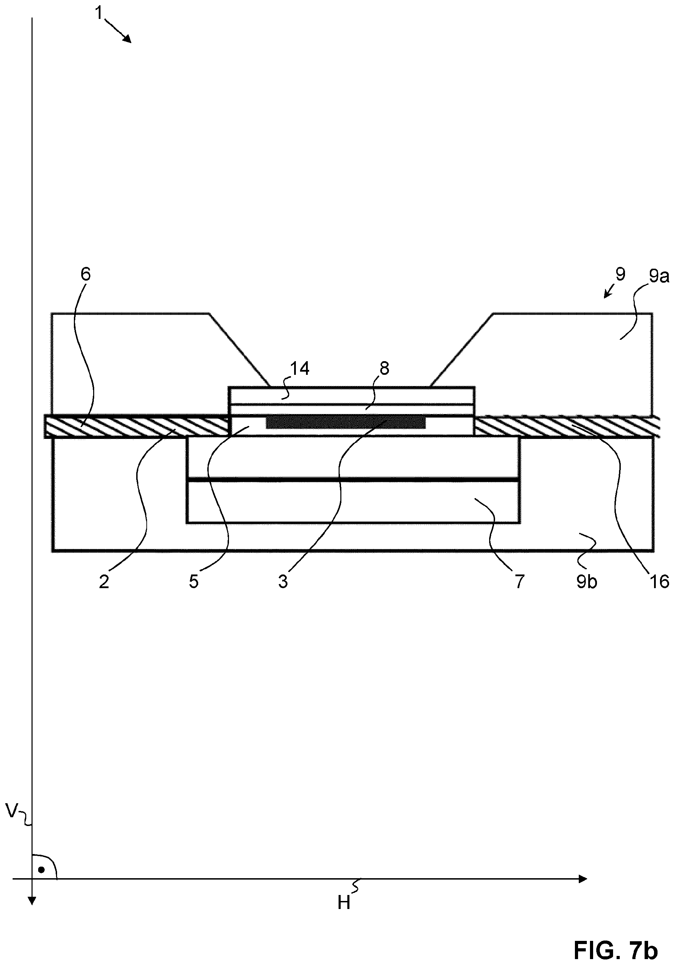

Fig. 7b shows a cross-sectional view of the assay device according to figure 7a;

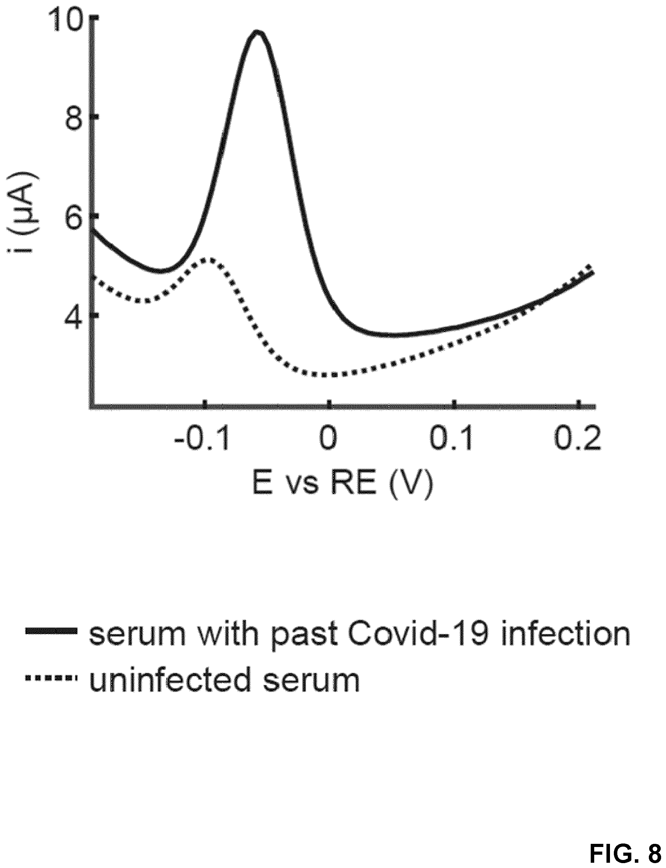

Fig. 8 shows experimental results using the assay device of figures 7a and 7b to measure Covid-19 serology in serum samples. Covid-19 nucleocapsid antigen are immobilized on a capture layer of the assay device and the anti-nucleocapsid IgG antibodies are detected in a sandwich configuration using a mouse anti-human secondary antibody labelled with alkaline phosphatase (ALP). The electroactive ALP product, 4-aminophenol, is detected at the electrode using square wave voltammetry;

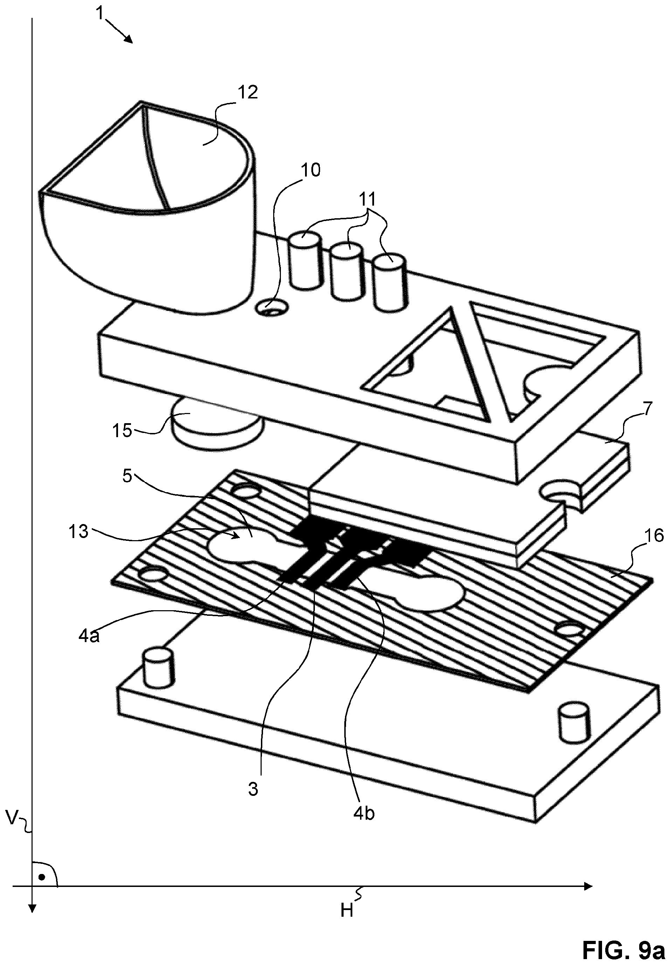

Fig. 9a shows an exploded view of the assay device in the form of a lateral flow assay device for flow injection analysis;

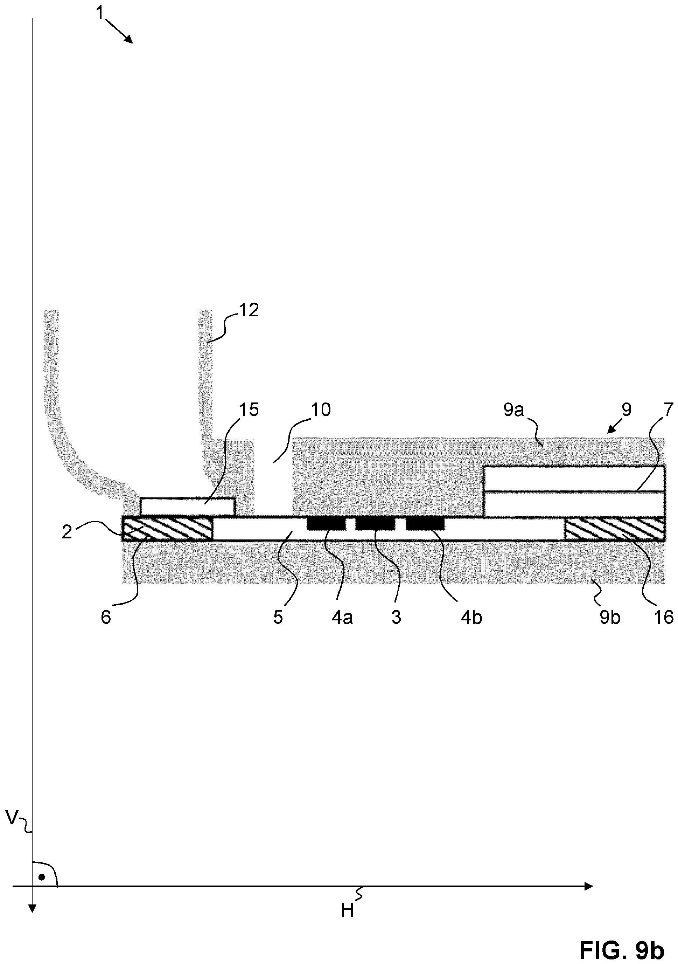

Fig. 9b show a cross-sectional view of the assay device according to figure 9a;

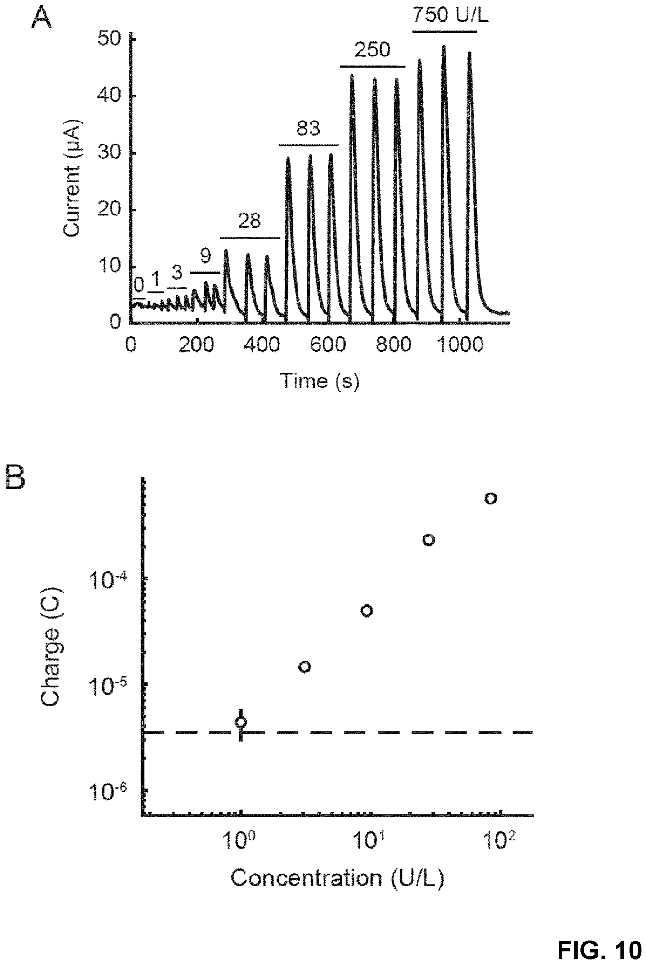

Fig. 10 shows continuous measurements of alkaline phosphatase samples (activity unit in U/L), an essential biomarker present in serum, using the assay device according to figures 9a and 9b. The samples are incubated off-chip with the enzyme substrate, injected in the assay device and detected downstream by the electrode system via chronoamperometry (constant voltage applied, current monitored over time). The integrated peak area (charge unit) is proportional to the concentration. More than 24 samples can be measured on the same device over 20 minutes.

## DESCRIPTION OF PREFERRED EMBODIMENTS

Various aspects of the assay devices 1 according to the invention will be explained in greater detail with respect to the figures.

As follows from figure 1, the assay devices 1 according to the invention comprise at least one substrate 2, at least one electrode 3, and at least one channel 5. The channel 5 extends at least partially in the substrate 2 and is configured to receive a sample fluid. A part of the electrode 3 is arranged in the channel 5 and is configured to detect at least one electrical property being associated with the sample fluid. The substrate 2, at least in the region of the channel 5, is porous. Here, the entire substrate 2 is porous and corresponds to a sheet of cellulose paper. At least the part of the electrode 3 being arranged in the channel 5 is porous. Here, the entire electrode 3 is porous and corresponds to a graphene material. Furthermore, in the depicted example the electrode 3 is entirely integrated into the substrate 2 and is generated by laser-pyrolysis of the substrate 2. The porous electrode 3 and the porous substrate 2 are configured to exert a capillary force onto the sample fluid in the channel 5 such that the sample fluid is flowing through the part of the electrode 3 being arranged in the channel 5. To this end it is preferred that at least the part of the electrode 3 being arranged in the channel 5 as well as that the substrate 2, in the region of the channel 5, are hydrophilic. In order to increase the hydrophilicity as well as the capillarity properties the electrode 3 is subject to an oxygen plasma treatment.

In order to delimit the channel 5 well, it is preferred that the substrate 2, in a region outside of the channel 5, is hydrophobic. Moreover, a part of the electrode 3 being arranged outside of the channel 5 is in the depicted example hydrophobic as well. To this end a coating 6 is provided, wherein the coating 6 is arranged on the substrate 2 and on the electrode 3 as well as in the substrate 2 and in the electrode 3. Said coating 6 at least partially delimits the channel 5 and is hydrophobic. Here, said coating corresponds to wax that is applied by lamination using a lamination device that comprises two rollers. Furthermore, in order to leave the channel 5 hydrophilic, no coating 6 is applied in the region of the channel 5. As such, a recess 13 in the form of a channel outline is removed from the coating 6 before the coating 6 is applied. Here, the channel outline is removed by irradiating laser radiation onto the coating 6 prior to its application.

In particular, and as indicated in figure 1, the manufacturing process comprises two main steps, pyrolysis and wax transfer, both of which rely here on the use of an optical source that emits radiation at a power in the range of 1 milliwatt to 50 watt, preferably in the range of 0.1 to 10 watt, most preferably in the range of 4 to 6 watt for patterning. The resulting functional layer or electrode layer 16 thus comprises a combination of one or more electrodes 3; 4a; 4b, and one or more channels 5 patterned independently from one another. That is, starting from a substrate 2 in the form of a cellulose paper layer (Whatman CF3 cellulose from Cytiva, 320 $\mu$m thickness) (panel A) that has been soaked with the fire retardant ammonium sulfamate ($NH_4SO_3NH_2$, 92 g/L, Sigma Aldrich) in order to prevent its combustion, and left to dry overnight, the electrode 3 is created and patterned (B) via laser-induced pyrolysis using a commercial $CO_2$ laser (Trotec Speedy 300) in engraving mode under nitrogen assist gas with the following settings: power 4.2 W, speed 16.4 cm/s, defocus 10 mm, engraving density 333 lines-per-inch. The material is then rinsed with water, isopropanol and acetone to remove salt residues and undesired soluble byproducts. Subsequently, the electrodes 3; 4a; 4b are treated by oxygen plasma (Diener Atto 100W plasma system) at 20-90% power and 1 mbar for 90 s.

A coating 6 in the form of a hydrophobic wax pattern is then transferred onto the paper substrate 2 via lamination to create the channel 5 (C). To this end a thin layer of paraffin wax (Xerox Cyan solid ink) was deposited on a polyester film (Folex X-100) using a wax printer (Xerox Colorqube 8570), and the hydrophilic recess 13 in the form of the outline of the channel 5 was cut off using the $CO_2$ laser. It is noted here that a commercially available wax printer has been used, although any other wax-coated film would likewise work. The wax patterns are contacted on both sides (asymmetric or symmetric design) of the cellulose layer and laminated at 110°C (3 passes) to transfer wax onto the paper/electrode composite layer. The resulting electrode layer is stored in a desiccator. Prior to the fabrication process, all designs (electrodes 3; 4a; 4b and channels 5) are generated digitally using Adobe Illustrator CS6.

Figure 2 confirms the successful pyrolysis of the cellulose substrate 2 into electrodes 3; 4a; 4b consisting of a conductive graphenic material by measuring the sheet resistance with the widely-used four-probe method in a square configuration (2 mm spacing), see panel A of figure 2. The voltage (E) is swept from -5 to 5 mV and the current (i) monitored using a potentiostat (Metrohm Autolab PGSTAT204). The sheet resistance $R_s$ (in $\Omega$/sq) is calculated as [1]:

$$R_s = \frac{E}{i} \cdot \frac{2\pi}{\ln(2)}$$

The formation of graphenic carbon structure ($sp^2$-bonded carbon sheets) [2] is attested by Raman spectroscopy, see Panel B of figure 2. The G-band at 1580 $cm^{-1}$ is characteristic of $sp^2$-bonded carbon material, and the D-band at 1340 $cm^{-1}$ stems from structural defects at the edges of the graphenic layers [3]. The 2D overtone at 2670 $cm^{-1}$ is strongly dependent on the stacking of the layers and would not be present in amorphous carbon [3].

The porosity of the pyrolyzed electrode material is evaluated by mercury porosimetry, see figure 3. The bottom layer of the cellulose paper substrate 2 is blocked by the coating 6 in the form of the wax (only the top half of the paper substrate 2 is pyrolyzed) to measure the electrode material only. Mercury (a non-wetting liquid) is contacted with the sample fluid at an increasing pressure and its absorbed volume precisely monitored by the porosimeter (Micromeritics Autopore IV). The amount of pressure required to force the mercury into the pores is inversely proportional to their diameters. The measurement is performed on 22 cm$^2$ of material (approximately 0.3 g) from a pressure of 3 kPa to 225 MPa which enables to investigate pore diameters ranging from 370 micrometer down to 5 nanometer (meso-/macro-porosity range). The pore diameter distribution is depicted against the absorption volume (normalized by the material surface area, in $\mu$L/cm$^2$) for each pore diameter.

Having assessed the porosity of the electrode materials, their capillary flow properties were evaluated from wicking experiments using a dye solution for contrast (6 mg/mL allura red in water, Sigma Aldrich). The paper strip (in the case of an assay device 1 in the form of a lateral flow assay device, see figures 9a and 9b), or stack of paper discs (for an assay device 1 in the form of a vertical flow assay device, see figures 7a and 7b) were contacted with the sample fluid on one of their extremities, and the flow front extracted from a video feed via thresholding (see figures 4a to 5b). The capillary flow through a porous medium follows the general Washburn equation

$$L = (D \cdot t)^{0.5}$$

Where L is the front position, t the time, and D the general Washburn coefficient (units: cm2/s) [4, 5]. This equation is most often used in the ideal case of linear pores with radius r, with $D = (\gamma/\eta) \cdot (r\cos(\theta))/2$, where $\gamma$ and $\eta$ are the surface tension and viscosity of the liquid, respectively, $\theta$ the contact angle of the liquid with the material. In the case of laser-pyrolyzed cellulose, with pore diameters similar to that of cellulose, one expects the capillary flow properties to strongly depend on the hydrophilicity of the material.

The electroactive area, and electrochemical porosity are derived from cyclic voltammetry experiments, see figures 6a to 6c. The voltammetry experiments are performed using a solution of 10 mM [Fe(CN)$_6$]$^{3-/4-}$ (Sigma Aldrich) in 1 M KCl at scan rates of 80, 50, 30 and 12 mV/s from -0.25 V to 0.25 V using a potentiostat (Metroohm Autolab PGSTAT204). The electroactive area is extracted from the slope of the peak current values $i_p$ plotted against the square root of the scan rate $v$, according to the Randles-Ševčík equation for diffusion-controlled reversible system [6,7]:

$$i_p = 2.69 \cdot 10^5 \ n^{3/2} A \ D^{1/2} C \ v^{1/2}$$

Where $n$ is the number of electrons transferred, A the electroactive area (in cm$^2$), D the diffusion coefficient (in cm$^2$/s), C the redox probe concentration (in mol/cm$^3$) and $v$ the scan rate (in V/s). The electrochemical porosity factor is calculated by dividing the electroactive area by the geometric (i.e. projected) area of the design.

While the conductive, (electro-)chemical properties of laser-pyrolyzed cellulose have been investigated in details [8, 9], their wetting properties have remained unexplored and unexploited. In the case of polyimide, the wetting properties can be tuned with the assist gas during lasing, from hydrophobic in oxygen-free conditions to hydrophilic in air or oxygen [10]. In the case of cellulose, a hydrophilic behaviour of the pyrolyzed material was observed by the inventors even when lasing in oxygen-free conditions, which together with its porous properties, open the door to capillary-driven bioassays.

For the electrochemical experiments, electrodes 3; 4a; 4b are patterned to form a 2- or 3-electrode system. In the case of a three-electrode system 3; 4a; 4b that has been used throughout the figures depicted here, the third electrode 4b is uncoated and used as a pseudo-reference electrode 4b. Voltages are thus reported against the pseudo reference electrode 4b (bare graphenic material), and not against a standard reference electrode material such as silver/silver chloride (Ag/AgCl). The fabrication process is thus greatly simplified at the expense of electrochemical accuracy (peak positions could shift depending on the composition of the solution).

As follows from figures 7a, 7b and 9a, 9b, the assay devices 1 according to the invention extend along a horizontal direction H as well as along a vertical direction V running perpendicularly to the horizontal direction H. Furthermore, the assay devices 1 comprise two main components: the electrode layer or functional layer 16 in the form of the substrate 2 with the integrated electrodes 3: 4a, 4b described above and an absorbent pad 7 to drive the flow (here 2 layers of Whatman CF6 cellulose from Cytiva). As mentioned above, the assay devices 1 comprise here a 3-electrode system comprising one working electrode 3, one counter electrode 4a and one pseudo-reference electrode 4b that is configured to establish a reference electrical potential for the electrical potential of the working electrode 3. In the assay device 1 of figures 7a and 7b, the electrodes 3; 4a; 4b are arranged at least partially around a circumferential direction of the channel 5 and extend from the substrate 2 partially into the channel 5. In the assay device 1 of figures 9a and 9b, the electrodes 3; 4a; 4b are arranged one after the other along the horizontal direction H of the assay device 1 and extend from one side of the channel 5 entirely across the channel 5. In figures 7a and 7b, the absorbent pad 7 is arranged after the substrate 2 when seen along the vertical direction V of the assay device 1 and is configured to absorb sample fluid flowing through the channel 5 along the vertical direction V of the assay device 1. Furthermore, the absorbent pad 7 is arranged congruent with the channel 5 when seen along the vertical direction V and at a same horizontal position as the channel 5. Furthermore, a cross-section of the absorbent pad 7 is larger than a cross-section of the channel 5 along the horizontal direction V. In figures 9a and 9b, however, the absorbent pad 7 is arranged before the substrate 2 when seen along the vertical direction V of the assay device 1. Furthermore, the absorbent pad is arranged in an end region of the channel 5 and extending beyond the channel 5 along the functional layer or electrode layer 16.

The major difference between the assay device 1 depicted in figures 7a and 7b and the assay device 1 depicted in figures 9a and 9b lies in the flow format. Namely, whereas figures 7a and 7b depict an assay device 1 in the vertical flow format, i.e. a vertical-flow immunoassay device, figures 9a and 9b depict an assay device 1 in the lateral flow format, i.e. a lateral-flow injection analysis device.

As follows from figures 7a and 7b, in the vertical-flow assay device 1 the channel 5 extends along the vertical direction V of the assay device 1, and wherein the substrate 2 is configured to exert the capillary force onto the sample fluid in the channel 5 such that the sample fluid is flowing along a vertical flowing direction Fv running parallel to the vertical direction V of the assay device 1. In the lateral-flow assay device 1 of figures 9a and 9b, however, the channel 5 extends along the horizontal direction H of the assay device 1, and wherein the substrate 2 is configured to exert the capillary force onto the sample fluid in the channel 5 such that the sample fluid is flowing along a horizontal flowing direction Fh running parallel to the horizontal direction H of the assay device 1.

Furthermore, the assay device 1 depicted in figures 7a and 7b comprise here a capture zone comprising capture molecules being configured to specifically capture at least one target property being associated with the sample fluid, wherein said capture zone is provided as a capture pad 8 being functionalized with the capture molecules. Said assay device 1 additionally comprises at least one sample pad 14 that is arranged before the substrate 2 when seen along the vertical direction V of the assay device 1. The sample pad 14 serves the purpose of protecting components of the assay device 1 being arranged after the sample pad 14 when seen along the vertical direction V of the assay device 1 and furthermore homogenises the wetting of the channel 5 across the whole cross-section.

The assay device 1 of figures 9a and 9b however comprises at least one buffer pad 15, wherein the buffer pad 15 is at least partially arranged in contact with the channel 5 and is configured to establish a steady flow of a buffer solution from a buffer reservoir 12 of the assay device 1 across the substrate 2 and to the absorbent pad 7. The buffer pad 15 furthermore serves the purpose of providing a wetting interface between the buffer reservoir 12 and the channel 5. As follows from figures 9a and 9b, the assay devices 1 can comprises a buffer reservoir 12 being configured to receive a buffer solution that is provided on a casing 9 of the assay device 1 (it is noted that the assay device 1 of figures 7a and 7b can likewise comprise such a buffer reservoir 12). In these figures the assay device 1 is depicted in the lateral flow format being configured for a flow injection analysis using a direct (unlabelled) detection of the sample fluid.

Furthermore, the assay devices 1 are preferably held together using casing 9. The casing 9 is preferably 3D-printed and encases the substrate 2, the electrodes 3, 4a; 4b and the channel 5 as well as further components of the assay device 1 such as the absorbent pad 7, the sample pad 14, and the buffer pad 15. Moreover, the casing 9 corresponds here to a two-part piece that comprises a top casing 9a and a bottom casing 9b that are arranged opposite to one another with respect to the horizontal direction H of the assay device 1. The casing 9 furthermore comprises electrode contacts 11 in the form of integrated spring contacts. The electrode contacts 11 are in electrical connection with the electrodes 3, 4a; 4b and furthermore with an evaluation device (not shown) for read-out and being configured to evaluate the detected electrical property. In addition, the casing 9 comprises a sample inlet 10 through which the sample fluid is insertable into the channel 5.

For the sake of completeness, the concrete design of a conceivable assay device 1 configured as a vertical-flow immunoassay device and of a conceivable assay device 1 configured for a lateral-flow injection analysis is discussed below.

In fact, figures 7a and 7b depict a vertical-flow assay device 1 comprising when seen the vertical direction V of the assay device 1 a top casing 9a with spring electrode contacts 11 and sample inlets 10, a cellulose sample pad 14 to avoid contamination of the capture pad 8, the capture pad 8 in the form of a nitrocellulose capture layer with immobilized protein (antigen or antibody), and the electrode layer or functional layer 16 comprising the substrate 2 with the integrated electrode system comprising two electrodes 3, 4a and a pseudo-reference electrode 4b with isolated electrode contacts 11 as well as a vertical fluidic channel 5, followed by a thick absorbent pad 7 to drive the capillary-driven flow after the wetting phase, and a bottom casing 9b with alignment pillars.

In particular, a nitrocellulose layer (BA85 nitrocellulose, pore diameter 400 nm from Cytiva) is used as the capture pad 8, wherein immobilization is performed by drop casting 3 $\mu$L of the protein solution (concentration: 0.1 to 1 mg/mL) onto a nitrocellulose disc of 7.5 mm diameter. The capture layers are left to dry overnight and stored at room temperature. All paper-based components (absorbent pad 7, capture pads 8) are cut to size using the $CO_2$ laser, enabling the precise control of the absorption volume of each component. For instance, the assay device 1 can be engineered to have a total absorption volume of 285 microliter, so that after addition of the last solution (enzyme substrate), the assay device 1 reaches a fully saturated state and can be incubated without any flow. This passive stop of the flow is another key feature of the assay device 1 according to the invention and a significant improvement over other approaches which require a manipulation to stop the flow during amplification.

The affinity of the capture zone such as the antibody/antigen affinity binding is leveraged to label a target property or target of interest. For instance and as illustrated in figure 8, in the event of the assay device 1 being configured as a SARS-CoV-2 serological immunoassay, the capture pad 8 in the form of the nitrocellulose layer is preferably functionalized with COVID-19 nucleocapsid antigen (LA612 from EastCoastBio). The assay can then be performed by adding the various solutions sequentially through the sample inlet 10 provided in the top casing 9a: First, 50 microliter of a blocking solution (calf serum, C8056 from Sigma, with 0.1% Tween20 and 10 mM Tris buffer pH 7.5) is pipetted into the assay device 1, followed by 25 microliter of sample fluid and then 25 microliter of the labelled secondary antibody (mouse anti-Human IgG conjugated to alkaline phosphatase, A2064 from Sigma) at a 1500-fold dilution in the blocking solution. Unbound species are then rinsed by 130 microliter of rinsing buffer (10 mM citrate buffer pH 3 with 0.1% Tween20), and the amplification performed by adding 120 microliter of substrate solution (3 mM 4-aminophenyl phosphate in 100 mM Tris buffer pH 9.8 and 20 mM $MgCl_2$). After 10 minutes, the signal being generated by the electrode 3 of the assay 1 device is read via square wave voltammetry (from -0.2 V to 0.25 V, step 5 mV, amplitude 25 mV, frequency 2.5 Hz) using the potentiostat (Metroohm Autolab PGSTAT204). In figure 8, the positive sample is a pooled human serum sample from patients with antibodies against SARS-CoV-2 (EURM017, Sigma) at a 2.5-fold dilution factor, and the negative sample is lab-grade sterile human serum (H4522 from Sigma).

The lateral-flow assay device 1 depicted in figures 9a and 9b comprise when seen along the vertical direction V of the assay device 1 a top casing 9a comprising a buffer reservoir 12, a sample inlet 10 in the form of a sample injection port and the electrode spring contacts 11. A steady flow is established from the buffer pad 15 to the absorbent pad 7 across the electrode layer or functional layer 16 comprising the substrate 2 with the integrated electrodes 3, 4a; 4b and the fluidic channel 5. The bottom casing 9b seals the assay device 1 and aligns the different layers or pads of the assay device 1 with its pillars.

Said lateral-flow assay device 1 comprises here a casing 9 that contains a buffer reservoir 12 on one side, and space for the absorbent pad 7 on the other side of the paper fluidic channel 5. The assay device 1 can be used for flow injection analysis, i.e. the sequential analysis of multiple samples spiked into a continuously running carrier buffer, see figure 10. In fact, and as follows from figure 10, for the detection of alkaline phosphatase, fluid samples containing alkaline phosphatase are mixed with the enzyme substrate (4-aminophenyl phosphate, p-APP) to reach a concentration of 10 mM p-APP, before incubation at 37 °C for 30 minutes in a thermoshaker. A volume of 5 microliter of each sample fluid is then injected into a carrier buffer (20 mM Tris buffer pH 7.5 with 0.4 M KCl, 0.1 % Tween 20 and 5 % calf serum C8056 from Sigma) at an interval ranging from 30 seconds to 5 minutes (depending on the current decay from the previous sample). In figure 10, the limit of detection is tested by measuring buffer samples (10 mM Tris buffer pH 7.5 with 1 % bovine serum albumin) with spiked alkaline phosphatase (P6774 from Sigma) with increasing concentrations. Detection is achieved by chronoamperometry with a constant applied voltage of 0.25 V and the current monitored over time by the potentiostat. The signal is calculated by integrated the peak area. Panel B of figure 10 shows the signals from triplicate measurements and the dashed line represents the minimum detectable signal (blank signal + 3 standards deviations).

Hence, in summary it is noted that the use of pyrolyzed electrodes in capillary-driven assay devices has been shown, harnessing the passive flow to perform a multistep assay or continuous analysis. Combined with the porous and permeable electrodes, a much simpler assay device for performing electrochemical immunoassay in a vertical flow format has been shown. Furthermore, the permeable electrodes of the assay device in the lateral flow format improve the signal strength due to a larger electroactive area, wherein the flow injection analysis has been used as an exemplar of a lateral flow system with permeable electrodes.

[1] I. Miccoli, F. Edler, H. Pfnür, C. Tegenkamp, "The 100th anniversary of the four-point probe technique: The role of probe geometries in isotropic and anisotropic systems" J. Phys. Condens. Matter 2015, 27, 223201.

[2] A. Bianco, H. M. Cheng, T. Enoki, Y. Gogotsi, R. H. Hurt, N. Koratkar, T. Kyotani, M. Monthioux, C. R. Park, J. M. D. Tascon, et al., "All in the graphene family - A recommended nomenclature for two-dimensional carbon materials" Carbon N. Y. 2013, 65, 1-6.

[3] D. B. Schuepfer, F. Badaczewski, J. M. Guerra-Castro, D. M. Hofmann, C. Heiliger, B. Smarsly, P. J. Klar, "Assessing the structural properties of graphitic and non-graphitic carbons by Raman spectroscopy" Carbon N. Y. 2020, 161, 359-372.

[4] R. L. Peek, D. A. Mclean, Capillary Penetration of Fibrous Materials, UTC, 1934.

[5] E. W. Washburn, "The dynamics of capillary flow" Phys. Rev. 1921, 17, 273-283.

[6] A. Ševčík, "Oscillographic polarography with periodical triangular voltage" Collect. Czechoslov. Chem. Commun. 1948, 13, 349-377.

[7] J. E. B. Randles, "A cathode ray polarograph. Part II. - The current-voltage curves" Trans. Faraday Soc. 1948, 44, 327-338.

[8] W. R. de Araujo, C. M. R. Frasson, W. A. Ameku, J. R. Silva, L. Angnes, T. R. L. C. Paixão, "Single-Step Reagentless Laser Scribing Fabrication of Electrochemical Paper-Based Analytical Devices" Angew. Chemie - Int. Ed. 2017, 56, 15113-15117.

[9] T. Pinheiro, S. Silvestre, J. Coelho, A. C. Marques, R. Martins, M. G. F. Sales, E. Fortunato, "Laser-Induced Graphene on Paper toward Efficient Fabrication of Flexible, Planar Electrodes for Electrochemical Sensing" Adv. Mater. Interfaces 2021, 8, 2101502.

[10] Y. Li, D. X. Luong, J. Zhang, Y. R. Tarkunde, C. Kittrell, F. Sargunaraj, Y. Ji, C. J. Arnusch, J. M. Tour, "Laser-Induced Graphene in Controlled Atmospheres: From Superhydrophilic to Superhydrophobic Surfaces" Adv. Mater. 2017, 29, 1700496.

LIST OF REFERENCE SIGNS

| | | | |
|---|---|---|---|
| 1 | assay device | 11 | electrode contact |
| 2 | substrate | 12 | buffer reservoir |
| 3 | electrode | 13 | recess |
| 4a, 4b | further electrode | 14 | sample pad |
| 5 | channel | 15 | buffer pad |
| 6 | coating | 16 | functional layer |
| 7 | absorbent pad | | |
| 8 | capture pad | H | horizontal direction |
| 9 | casing | V | vertical direction |
| 9a | top casing | Fh | horizontal flowing direction |
| 9b | bottom casing | Fv | vertical flowing direction |
| 10 | sample inlet | | |

**Claims**

1. An assay device (1) for electrochemical sensing of a sample fluid comprising:

   - at least one substrate (2),
   - at least one electrode (3),
   - at least one channel (5),

   wherein the substrate (2) extends along a vertical direction (V) of the assay device (1) and along a horizontal direction (H) of the assay device (1) running perpendicularly to the vertical direction (V),
   wherein the channel (5) extends at least partially in the substrate (2) and is configured to receive the sample fluid,
   wherein at least part of the electrode (3) is arranged in the channel (5) and is configured to detect at least one electrical property being associated with the sample fluid,
   **characterized in that** the substrate (2), at least in the region of the channel (5), is porous, and
   **in that** at least the part of the electrode (3) being arranged in the channel (5) is porous, and
   wherein the substrate (2) is configured to exert a capillary force onto the sample fluid in the channel (5) such that the sample fluid is flowing through the part of the electrode (3) being arranged in the channel (5).

2. The assay device (1) according to claim 1, wherein the electrode (3) is at least partially and preferably entirely integrated into the substrate (2), and/or
   wherein the electrode (3) is generated by pyrolysis of the substrate (2), in particularly by laser-pyrolysis or by flash pyrolysis or by using a pyrolysis furnace.

3. The assay device (1) according to any one of the preceding claims, wherein the substrate (2) comprises or consists of at least one of a paper-based compound or a polymeric membrane material, the paper-based compound preferably comprising cellulose fibers and/or the polymeric membrane material preferably being nitrocellulose or polysulfone, and/or
wherein the electrode (3) comprises or consists of at least one of a carbonaceous compound or a paper-templated metal, the carbonaceous compound preferably being a graphenic material.

4. The assay device (1) according to any one of the preceding claims, wherein at least the part of the electrode (3) being arranged in the channel (5) is hydrophilic, and/or
wherein a part of the electrode (3) is arranged outside of the channel (5), said part of the electrode (3) being hydrophobic.

5. The assay device (1) according to any one of the preceding claims, wherein, in the region of the channel (5), the substrate (2) is hydrophilic, and/or
wherein, in a region outside of the channel (5), the substrate (2) is hydrophobic.

6. The assay device (1) according to any one of the preceding claims, further comprising at least one coating (6),

wherein the coating (6) is at least partially arranged on and/or in at least one of the substrate (2) or the electrode (3), and/or
wherein the coating (6) at least partially delimits the channel (5), and/or wherein the coating (6) is at least one of hydrophobic, has a melting temperature of 150° or less, or comprises or consists of wax, preferably paraffin.

7. The assay device (1) according to any one of the preceding claims, wherein the channel (5) extends along the vertical direction (V) of the assay device (1), and
wherein the substrate (2) is configured to exert the capillary force onto the sample fluid in the channel (5) such that the sample fluid is flowing along a vertical flowing direction (Fv) running parallel to the vertical direction (V) of the assay device (1).

8. The assay device (1) according to any one of the preceding claims, wherein the channel (5) extends along the horizontal direction (H) of the assay device (1), and
wherein the substrate (2) is configured to exert the capillary force onto the sample fluid in the channel (5) such that the sample fluid is flowing along a horizontal flowing direction (Fh) running parallel to the horizontal direction (H) of the assay device (1).

9. The assay device (1) according to any one of the preceding claims, further comprising at least one absorbent pad (7) being arranged before and/or after the substrate (2) when seen along the vertical direction (V) of the assay device (1), and
wherein the absorbent pad (7) is configured to absorb the sample fluid.

10. The assay device (1) according to any one of the preceding claims, further comprising at least one capture zone comprising capture molecules such as antibodies being configured to specifically capture at least one target property being associated with the sample fluid, and

wherein the capture zone is provided as a capture pad (8) being functionalized with the capture molecules, and/or
wherein the capture zone is provided by the electrode (3) being functionalized with the capture molecules.

11. The assay device (1) according to any one of the preceding claims, wherein the electrode (3) is a working electrode and wherein the assay device further comprises at least one counter electrode (4a), and wherein an electrical potential or current is applicable and/or measurable between the working electrode and the counter electrode, and

wherein the assay device preferably further comprises at least one reference electrode (4b) being configured to establish a reference electrical potential, wherein the electrical potential being applicable and/or measurable at the working electrode is established with respect to the reference potential and/or
further comprising at least one evaluation device being configured to evaluate the electrical property being detected by the electrode (3).

12. The assay device (1) according to any one of the preceding claims, further comprising a casing (9), and

wherein the casing at least partially encases the substrate (2), the electrode and the channel (5), and/or
wherein the casing (9) comprises at least one of:

- at least one sample inlet (10) through which the sample fluid is insertable into the channel (5),
- at least one electrode contact (11) being in electrical connection with the electrode (3) and being configured to transmit an electrical signal of the electrode (3) being associated with the detected electrical property to an evaluation device, or
- at least one buffer reservoir (12) being configured to receive a buffer solution.

13. A method of manufacturing an assay device (1) for electrochemical sensing of a sample fluid, preferably the assay device (1) as claimed in any one of the preceding claims, the method comprising:

- Providing at least one substrate (2),
- Providing at least one electrode (3),
- Providing at least one channel (5),

wherein the substrate (2) extends along a vertical direction (V) of the assay device (1) and along a horizontal direction (H) of the assay device (1) running perpendicularly to the vertical direction (V),
wherein the channel (5) extends at least partially through the substrate (2) and is configured to receive the sample fluid,
wherein at least part of the electrode (3) is arranged in the channel (5) and is configured to detect at least one electrical property being associated with the sample fluid,
**characterized in that** the substrate (2) at least in the region of the channel (5) is porous, and **in that** at least the part of the electrode (3) being arranged in the channel (5) is porous, and
wherein the substrate (2) is configured to exert a capillary force onto the sample fluid in the channel (5) such that the sample fluid is flowing through the part of the electrode (3) being arranged in the channel (5).

14. The method according to claim 13, wherein the electrode (3) is integrally formed in the substrate (2) by pyrolysis of the substrate (2), in particular by laser pyrolysis or by flash pyrolysis or by using a pyrolysis furnace, and/or wherein the electrode (3) is treated with a plasma, in particular an oxygen plasma and/or an air plasma.

15. The method according to claim 13 or 14, wherein at least one coating (6) is provided, and wherein a recess (13) in the form of a channel outline is removed from the coating (6) preferably by irradiating laser radiation onto the coating (6), and
wherein the coating (6) is preferably applied on and/or in the substrate (2) and/or the electrode (3) after the recess (13) in the form of the channel outline is formed and/or by lamination.

FIG. 1

paper
LPE

FIG. 2

FIG. 3

**FIG. 4a**

**FIG. 4b**

**FIG. 5a**

**FIG. 5b**

**FIG. 6a**

**FIG. 6b**

FIG. 6c

FIG. 7a

FIG. 7b

— serum with past Covid-19 infection

······ uninfected serum

FIG. 8

FIG. 9a

FIG. 9b

FIG. 10

Europäisches
Patentamt

European
Patent Office

Office européen
des brevets

# EUROPEAN SEARCH REPORT

Application Number

EP 22 18 6139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 109 060 923 A (UNIV ZHEJIANG) 21 December 2018 (2018-12-21) | 1-11,13 | INV.<br>G01N27/327 |
| Y | * abstract *<br>* paragraphs [0037] - [0047]; figures 1-3 * | 14 | G01N33/543 |
| X | WO 2015/138978 A1 (UNIV TEXAS [US]) 17 September 2015 (2015-09-17)<br>* page 14, line 21 - page 36, line 29; figures 1-3,10, 15-16, 26 * | 1-9, 11-13,15 | |
| X | EP 3 514 542 A1 (UNIV SOUTHERN TAIWAN SCI & TEC [TW]) 24 July 2019 (2019-07-24)<br>* paragraphs [0011] - [0012]; figures 1-7 * | 1-13,15 | |
| X | WO 2013/036617 A1 (HARVARD COLLEGE [US]; WHITESIDES GEORGE M [US] ET AL.) 14 March 2013 (2013-03-14)<br>* paragraphs [0078] - [0099]; figure 1 * | 1-11,13 | |
| X | WO 2010/102279 A1 (HARVARD COLLEGE [US]; NEI ZHIHONG [US] ET AL.) 10 September 2010 (2010-09-10)<br>* paragraphs [0052] - [0067]; figures 1-3 * | 1-8, 10-13 | TECHNICAL FIELDS SEARCHED (IPC)<br><br>G01N |
| Y | EP 3 951 374 A1 (CONSEJO SUPERIOR INVESTIGACION [ES]) 9 February 2022 (2022-02-09)<br>* paragraphs [0001], [0029] - [0073]; figures 1-6 * | 1-13,15 | |
| Y | WO 2004/061418 A2 (MESO SCALE TECHNOLOGIES LLC [US]; GLAZER ELI N [US] ET AL.) 22 July 2004 (2004-07-22)<br>* page 42, lines 15-22 * | 1-13,15 | |

-/--

The present search report has been drawn up for all claims

| Place of search | Date of completion of the search | Examiner |
|---|---|---|
| Munich | 13 January 2023 | Lazar, Zala |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 22 18 6139

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NOVIANA EKA ET AL: "Microfluidic Paper-Based Analytical Devices: From Design to Applications", CHEMICAL REVIEWS, vol. 121, no. 19, 13 October 2021 (2021-10-13), pages 11835-11885, XP055854824, US ISSN: 0009-2665, DOI: 10.1021/acs.chemrev.0c01335 * page 11854, column 2, paragraph 3 * ----- | 14 | |

TECHNICAL FIELDS
SEARCHED (IPC)

The present search report has been drawn up for all claims

| Place of search | Date of completion of the search | Examiner |
|---|---|---|
| Munich | 13 January 2023 | Lazar, Zala |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 22 18 6139

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2023

| Patent document cited in search report | | | Publication date | Patent family member(s) | | Publication date |
|---|---|---|---|---|---|---|
| CN 109060923 | A | | 21-12-2018 | NONE | | |
| WO 2015138978 | A1 | | 17-09-2015 | US | 2017173578 A1 | 22-06-2017 |
| | | | | WO | 2015138978 A1 | 17-09-2015 |
| EP 3514542 | A1 | | 24-07-2019 | NONE | | |
| WO 2013036617 | A1 | | 14-03-2013 | US | 2014183059 A1 | 03-07-2014 |
| | | | | WO | 2013036617 A1 | 14-03-2013 |
| WO 2010102279 | A1 | | 10-09-2010 | AU | 2010221102 A1 | 29-09-2011 |
| | | | | CA | 2754577 A1 | 10-09-2010 |
| | | | | EP | 2403645 A1 | 11-01-2012 |
| | | | | ES | 2612507 T3 | 17-05-2017 |
| | | | | PL | 2403645 T3 | 31-05-2017 |
| | | | | US | 2012181184 A1 | 19-07-2012 |
| | | | | WO | 2010102279 A1 | 10-09-2010 |
| EP 3951374 | A1 | | 09-02-2022 | EP | 3951374 A1 | 09-02-2022 |
| | | | | WO | 2022029013 A1 | 10-02-2022 |
| WO 2004061418 | A2 | | 22-07-2004 | AU | 2003302263 A1 | 29-07-2004 |
| | | | | AU | 2011200010 A1 | 27-01-2011 |
| | | | | CA | 2511389 A1 | 22-07-2004 |
| | | | | CA | 2772050 A1 | 22-07-2004 |
| | | | | CA | 2941139 A1 | 22-07-2004 |
| | | | | CA | 3122193 A1 | 22-07-2004 |
| | | | | CA | 3171720 A1 | 22-07-2004 |
| | | | | CN | 101098956 A | 02-01-2008 |
| | | | | CN | 102620959 A | 01-08-2012 |
| | | | | EP | 1583950 A2 | 12-10-2005 |
| | | | | EP | 2711415 A2 | 26-03-2014 |
| | | | | HK | 1117189 A1 | 09-01-2009 |
| | | | | JP | 4764010 B2 | 31-08-2011 |
| | | | | JP | 5127878 B2 | 23-01-2013 |
| | | | | JP | 5367019 B2 | 11-12-2013 |
| | | | | JP | 2006517652 A | 27-07-2006 |
| | | | | JP | 2010243498 A | 28-10-2010 |
| | | | | JP | 2011169908 A | 01-09-2011 |
| | | | | JP | 2011203272 A | 13-10-2011 |
| | | | | US | 2004189311 A1 | 30-09-2004 |
| | | | | US | 2009065357 A1 | 12-03-2009 |
| | | | | US | 2009066339 A1 | 12-03-2009 |
| | | | | US | 2012055809 A1 | 08-03-2012 |
| | | | | US | 2014151224 A1 | 05-06-2014 |
| | | | | US | 2014378341 A1 | 25-12-2014 |

EPO FORM P0459

For more details about this annex : see Official Journal of the European Patent Office, No. 12/82

**page 1 of 2**

## ANNEX TO THE EUROPEAN SEARCH REPORT
## ON EUROPEAN PATENT APPLICATION NO.

EP 22 18 6139

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2023

| Patent document cited in search report | Publication date | Patent family member(s) | | Publication date |
|---|---|---|---|---|
| | | US | 2016356722 A1 | 08-12-2016 |
| | | US | 2018172595 A1 | 21-06-2018 |
| | | US | 2020355616 A1 | 12-11-2020 |
| | | US | 2021055287 A1 | 25-02-2021 |
| | | WO | 2004061418 A2 | 22-07-2004 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **I. MICCOLI ; F. EDLER ; H. PFNÜR ; C. TEGENKAMP.** The 100th anniversary of the four-point probe technique: The role of probe geometries in isotropic and anisotropic systems. *J. Phys. Condens. Matter,* 2015, vol. 27, 223201
- **A. BIANCO ; H. M. CHENG ; T. ENOKI ; Y. GOGOTSI ; R. H. HURT ; N. KORATKAR ; T. KYOTANI ; M. MONTHIOUX ; C. R. PARK ; J. M. D. TASCON et al.** All in the graphene family - A recommended nomenclature for two-dimensional carbon materials. *Carbon N. Y.,* 2013, vol. 65, 1-6
- **D. B. SCHUEPFER ; F. BADACZEWSKI ; J. M. GUERRA-CASTRO ; D. M. HOFMANN ; C. HEILIGER ; B. SMARSLY ; P. J. KLAR.** Assessing the structural properties of graphitic and non-graphitic carbons by Raman spectroscopy. *Carbon N. Y.,* 2020, vol. 161, 359-372
- **R. L. PEEK ; D. A. MCLEAN.** Capillary Penetration of Fibrous Materials. *UTC,* 1934
- **E. W. WASHBURN.** The dynamics of capillary flow. *Phys. Rev.,* 1921, vol. 17, 273-283

- **J. E. B. RANDLES.** A cathode ray polarograph. Part II. - The current-voltage curves. *Trans. Faraday Soc.,* 1948, vol. 44, 327-338
- **W. R. DE ARAUJO ; C. M. R. FRASSON ; W. A. AMEKU ; J. R. SILVA ; L. ANGNES ; T. R. L. C. PAIXÃO.** Single-Step Reagentless Laser Scribing Fabrication of Electrochemical Paper-Based Analytical Devices. *Angew. Chemie - Int. Ed.,* 2017, vol. 56, 15113-15117
- **T. PINHEIRO ; S. SILVESTRE ; J. COELHO ; A. C. MARQUES ; R. MARTINS ; M. G. F. SALES ; E. FORTUNATO.** Laser-Induced Graphene on Paper toward Efficient Fabrication of Flexible, Planar Electrodes for Electrochemical Sensing. *dv. Mater. Interfaces,* 2021, vol. 8, 2101502
- **Y. LI ; D. X. LUONG ; J. ZHANG ; Y. R. TARKUNDE ; C. KITTRELL ; F. SARGUNARAJ ; Y. JI ; C. J. ARNUSCH ; J. M. TOUR.** Laser-Induced Graphene in Controlled Atmospheres: From Superhydrophilic to Superhydrophobic Surfaces. *Adv. Mater.,* 2017, vol. 29, 1700496